# EUROPEAN PATENT SPECIFICATION

(11) **EP 2 307 567 B1**
(45) Date of publication and mention of the grant of the patent: **10.09.2014**
(21) Application number: 09768879.0
(22) Date of filing: 25.05.2009
(51) Int. Cl.: C12Q 1/68

(54) **METHODS FOR PRODUCING INDUCIBLE AND/OR REPRESSIBLE EXPRESSION ACTIVE LINEAR RNA INTERFERENCE CASSETTES AND INDUCIBLE AND/OR REPRESSIBLE EXPRESSION ACTIVE LINEAR GENE CASSETTES AND THEIR USES**
VERFAHREN ZUR HERSTELLUNG INDUZIERBARER UND/ODER REPRIMIERBARER EXPRESSIONSAKTIVER LINEARER RNA-INTERFERENZ-KASSETTEN SOWIE INDUZIERBARE UND/ODER REPRIMIERBARE EXPRESSIONSAKTIVE LINEARE GEN-KASSETTEN UND DEREN VERWENDUNGEN
PROCÉDÉS POUR PRODUIRE DES CASSETTES D INTERFÉRENCE ARN LINÉAIRES ACTIVES À EXPRESSION INDUCTIBLE ET/OU RÉPRESSIBLE ET CASSETTES DE GÈNE LINÉAIRES ACTIVES À EXPRESSION INDUCTIBLE ET/OU RÉPRESSIBLE ET LEURS UTILISATIONS

(30) Priority: 27.06.2008 WO PCT/EP2008/005278; 17.11.2008 WO PCT/EP2008/009712
(43) Date of publication of application: 13.04.2011
(73) Proprietor: King Faisal Specialist Hospital & Research Centre, Riyadh 11211 (SA)
(72) Inventor: KHABAR, Khalid, S., Abu, Riyadh 11211 (SA)
(74) Representative: Engelhard, Markus
(86) International application number: PCT/EP2009/003686
(87) International publication number: WO 2009/156030

(56) References cited:
- WO-A-01/21834
- WO-A-2004/013288
- WO-A-2005/095615
- WO-A-2006/081831
- SAMBROOK, J. AND RUSSELL D.: "Molecular Cloning" 2001, COLD SPRING HARBOR LABORATORY PRESS , COLD SPRING HARBOR , XP002497435 page 8.37 - page 8.45 page 9.36 - page 9.37 page 17.30 - page 17.51
- ROA-RODRIGUES, CAROLINA: "Promoters used to regulate gene expression"[Online] 11 April 2007 (2007-04-11), XP002538187 Retrieved from the Internet: URL:http://www.patentlens.net/daisy/promot ers/768.html> [retrieved on 2009-07-21]
- CASTANOTTO D ET AL: "Functional siRNA expression from transfected PCR products" RNA, COLD SPRING HARBOR LABORATORY PRESS, WOODBURY, NY, US, vol. 8, 1 January 2002 (2002-01-01), pages 1454-1460, XP002977882 ISSN: 1355-8382
- HE ET AL: "Interference of porcine reproductive and respiratory syndrome virus replication on MARC-145 cells using DNA-based short interfering RNAs" ANTIVIRAL RESEARCH, ELSEVIER SCIENCE BV., AMSTERDAM, NL, vol. 74, no. 2, 23 March 2007 (2007-03-23), pages 83-91, XP005933983 ISSN: 0166-3542

## Description

The present invention relates to a method for assessing post-transcriptional effects or for identifying compounds that affect post-transcriptional regulation of genes, comprising producing inducible and/or repressible expression active linear gene constructs comprising a PCR amplification of a source expression polynucleotide comprising a promoter sequence and the DNA sequence of interest

### BACKGROUND OF THE INVENTION

RNA interference (RNAi) is a system within living cells that helps to control which genes are active and how active they are. Two types of small RNA molecules - micro RNA (miRNA) and small interfering RNA (siRNA) - are central to RNA interference. RNAs are the direct products of genes, and these small RNAs can bind to specific other RNAs and either increase or decrease their activity, for example by preventing a messenger RNA (mRNA) from producing a protein. RNA interference has an important role in defending cells against parasitic genes but also in directing development as well as gene expression in general.

The RNAi pathway is found in many eukaryotes and is initiated by the enzyme Dicer which cleaves long double-stranded RNA (dsRNA) molecules into short fragments of ∼20 nucleotides. One of the two strands of each fragment, known as the guide strand, is then incorporated into the RNA-induced silencing comples (RISC). The most well-studied outcome is post-transcriptional gene silencing, which occurs when the guide strand base pairs with a complementary sequence of a messenger RNA molecule and induces cleavage by Argonaute, the catalytic component of the RISC complex. This process is known to spread systemically throughout the organism despite initially limited molar concentrations of siRNA.

The selective and robust effect of RNAi on gene expression makes it a valuable research tool, both in cell culture and in living organisms because synthetic dsRNA introduced into cells can induce suppression of specific genes of interest. RNAi may also be used for large-scale screens that systematically shut down each gene in the cell, which can help identify the components necessary for a particular cellular process or an event such as cell division. Exploitation of the pathway is also a promising tool in biotechnology and medicine.

RNA interference (RNAi) is a post-transcriptional gene-silencing mechanism, by which a specific degradation of mRNA is induced by homologous double-stranded RNAs (dsRNAs), and constitutes a powerful tool for gene function analysis.

RNAi can occur by introducing chemically synthesized siRNA or by endogenous microRNAs (miRNA) that silence cellular mRNAs (miRNAs). Reviewed in Sledz CA, Williams BR. 2005. RNA interference in biology and disease. Blood. 106(3):787-94).

The synthetic dsRNA of 21-23 nucleotides that target a specific region in the mRNA is a synthetic siRNA. Taken advantage of miRNA structure, short hairpin forms (e.g., expressed from shRNA vectors) are about 50-70 nucleotides that comprise sense strand, loop sequences, antisense strand and termination sequence. Once in cells, the hairpins are processed by dicer enzyme into an siRNA that mediates gene silencing. In mammalian cells, the antisense strand of synthetic short interfering RNA (siRNA) serves as a template for the RNA-induced silencing complex (RISC) to recognize and cleave complementary messenger RNA (mRNA), which is then rapidly degraded.

The short inhibitory RNAs (siRNAs) are commonly made as chemically synthesized double-stranded short RNA (19-29 mer) which is relatively easy and has a higher transfection rate. However, it is costly and transient in action. Vector based RNAi approaches included the use of short hairpin RNA (shRNA) as plasmid or virus-based vectors.

The need for inducible (regulated) RNAi vectors allow the precise and temporal control of gene knockdown, to avoid artifactual differences between compared cell populations, reduction of non-specific cellular effects due to induction of transient expression, and for expression of RNAi when extended can cause cellular toxicity or unwanted changes.

The problem is that once RNAi vectors are made, inclusion of regulated sequences, such as tetracycline operator (TetO) sites, require time consuming cloning and use of enzymes, such as restriction enzymes, ligase, topoisomerase, or recombinase. Though the TetO-sites can be synthesized as double stranded polynucleotides or as a PCR product, these still need to be cloned into the vector using restriction sites and cloning enzymes. All of these cloning-based techniques require propagation of the plasmid (including transformation into bacterial cells, culture growth, and plasmid extraction) or the virus-based vector include packaging in cells and virus purification. These cloning-based processes become almost formidable when working with large number of RNAi vectors such as in high-throughput applications.

WO 2005/095615 A1 describes an assay for the identification of biologically active compounds, in particular a reporter gene assay for the identification of compounds, which have an effect on mRNA stability. In particular, WO 2005/095615 A describes a reporter gene expression system and cell lines comprising said expression system as well as compounds which destabilize mRNA.

WO 01/21834 A1 describes a method for studying the effect of a polymorphism on the activity of a regulatory element such as a promoter. The method comprises the steps of providing a first adenoviral vector, comprising: (i) a regulatory element having a first nucleic acid sequence, the regulatory element being operably linked to a reporter gene; (ii) inserting the first adenoviral vector into a suitable host cell; and (iii) detecting a first value of reporter gene expression. This may be compared with the results from a second adenoviral vector which comprises a regulatory element having a different nucleic acid sequence. Alternatively, the first value of reporter gene expression may be compared with that obtained from a vector comprising the promoter-reporter construct attached to a 3'-UTR. This allows the effect of a 3'-UTR on a regulatory element polymorphism to be studied.

There is a need in the art to improve the methods of providing a (any) gene sequence of interest in an expressable (ready-to-use) format that is simple to handle and/or which furthermore allows assessing post-transcriptional effects.

### SUMMARY OF THE INVENTION

The object of the present invention is solved by the subject-matter as defined in the attached claims.

The object of the present invention is particularly solved by a method for assessing post-transcriptional effects or for identifying compounds that affect post-transcriptional regulation of genes, said method comprising a single PCR-based method that simultaneously generates a linear gene expression cassette that is also inducible/repressible, such as utilizing the TetO system or any another sequence that causes inducible/repressible expression when linked to an expression cassette.

In particular, the object of the present invention is solved by providing a method for assessing post-transcriptional effects or for identifying compounds that affect post-transcriptional regulation of genes, said method comprising producing inducible expression active linear gene constructs.

Said method comprises the steps of:
(a) producing an inducible expression active linear gene construct, comprising
   one or more control element(s) which are inducible (on/off) elements,
   a minimal promoter which is a minimal promoter derived from a ribosomal protein promoter,
   a DNA sequence selected from a gene, a coding region, an open reading frame (ORF) and a cDNA,
   a 3' untranslated region (3' UTR) containing mRNA destabilization or stabilization elements derived from a 3' UTR of a cellular mRNA, and
   a termination sequence,
   by a PCR amplification of a source expression polynucleotide comprising in 5' to 3' direction a promoter sequence and the DNA sequence using
   (i) a forward primer comprising
      at its 3' end, a first sequence part complementary to a promoter region of the source expression polynucleotide upstream of the DNA sequence, and
      at its 5' end a second sequence part comprising one or more introduced control element(s); and
   (ii) a reverse primer selected from
      a reverse primer complementary to a region of the source expression polynucleotide downstream of the DNA sequence comprising mRNA destabilization or stabilization elements derived from a 3' UTR of a cellular mRNA and a termination sequence, or
      a reverse primer complementary to a region of the source expression polynucleotide downstream of the 3' UTR wherein the region contains mRNA destabilization or stabilization elements of a cellular mRNA with or without a termination sequence,
   wherein the source expression polynucleotide is selected from a vector (like an expression vector), a lentivirus, a plasmid, a virus-based vector, or a linear or linearized or amplified fragment thereof,
   wherein said construct is obtained in a single step not involving a cloning step, wherein said cloning step includes the use of restriction digestion, cloning enzyme(s), bacterial transformation and/or plasmid preparation,
   wherein expression of the inducible expression active linear gene construct is connected with the inducibility of the promoter, which requires the addition of a compound which activates the expression and/or which requires the withdrawal of a compound which represses the expression, and
(b) assessing post-transcriptional effects at conditions that inhibit the inducible expression of the gene or protein due to the absence of a compound which activates the expression and/or the presence of a compound which represses the expression.

In particular, the object of the present invention is furthermore solved by providing a method for assessing post-transcriptional effects or for identifying compounds that affect post-transcriptional regulation of genes, said method comprising producing repressible expression active linear gene constructs.

Said method preferably comprises the step of:
(a) producing a repressible expression active linear gene construct, comprising
   one or more control element(s) which are repressible (on/off) element(s),
   a minimal promoter which is a minimal promoter derived from a ribosomal protein promoter,
   a DNA sequence selected from a gene, a coding region, an open reading frame (ORF) and a cDNA,
   a 3' untranslated region (3' UTR) containing mRNA destabilization or stabilization elements derived from a 3' UTR of a cellular mRNA, and
   a termination sequence,
   by a PCR amplification of a source expression polynucleotide comprising in 5' to 3' direction a promoter sequence and the DNA sequence using
   (i) a forward primer comprising
      at its 3' end, a first sequence part complementary to a promoter region of the source expression polynucleotide upstream of the DNA sequence, and
      at its 5' end a second sequence part comprising one or more introduced control element(s); and
   (ii) a reverse primer selected from
      a reverse primer complementary to a region of the source expression polynucleotide downstream of the DNA sequence comprising mRNA destabilization or stabilization elements derived from a 3' UTR of a cellular mRNA and a termination sequence, or
      a reverse primer complementary to a region of the source expression polynucleotide downstream of the 3' UTR wherein the region contains mRNA destabilization or stabilization elements of a cellular mRNA with or without a termination sequence,
   wherein the source expression polynucleotide is selected from a vector, a lentivirus, a plasmid, a virus-based vector, or a linear or linearized or amplified fragment thereof,
   wherein said construct is obtained in a single step not involving a cloning step, wherein said cloning step includes the use of restriction digestion, cloning enzyme(s), bacterial transformation and/or plasmid preparation,
   wherein expression of the repressible expression active linear gene construct is connected with the inducibility of the promoter, which requires the addition of a compound which represses the expression and/or which requires the withdrawal of a compound which activates the expression, and
(b) assessing post-transcriptional effects at conditions that inhibit the inducible expression of the gene or protein due to the absence of a compound which represses the expression and/or presence of a compound which activates the expression.

According to the present invention this object is furthermore solved by a method for identifying compounds that affect post-transcriptional regulation of reporter(s) or gene(s).

Said method preferably comprises the following steps
(a) providing at least one inducible/repressible expression active linear gene construct as defined according to the present invention, or a stable cell line harbouring an inducible/repressible expression active linear gene construct as defined according to the present invention expressing a reporter or gene protein from the construct,
(b) providing at least a compound to be tested,
(c) determining the effect of the compound on the post-transcriptional regulation by determining the mRNA level and/or the expression level of the reporter or gene.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### 1. Methods for producing inducible and/or repressible expression active linear RNA interference constructs (first aspect described in the present invention)

The present invention describes methods for producing inducible and/or repressible expression active linear ***DNA interference constructs.***

The ease of the methods in converting any RNA inhibitory expressing vector or expressable polynucleotide into TetO or other regulated constructs without virtually any cloning or plasmid preparation step is a remarkable feature describes in the disclosed invention.

The inducible/repressible expression active linear RNA interference constructs are ready to be used for expressing the RNA interference of interest in cells in a regulatable/regulated manner.

"RNA interference" (RNAi) refers to a post-transcriptional gene-silencing mediated by homologous double-stranded dsRNA inside cells. RNAi can occur by expressing inside cells RNA sequences which specifically target mRNAs of interest. These RNA sequences comprise for example short hairpin RNA (shRNA), small interfering RNA (siRNA), micro RNA (miRNA) or antisense RNA. RNAi can be used for gene silencing, gene knockdown, functional genomics, and therapeutic applications. When cells are exposed to double stranded RNA, which is either formed naturally through miRNA processing pathway or when expressed from an RNAi vector (such as shRNA vector), the dsRNA is then cleaved by a protein called Dicer. This will lead to formation of small 21-23 nucleotide siRNAs which associate with the specific mRNA targeted by their nucleotide sequence in a complex called RISC, which includes RNase activity that degrades the target mRNA. Synthetic siRNA can also be directly transfected into cells to mediate silencing.

Said method described herein comprises the step of:
(a) generating an inducible/repressible expression active linear RNA interference construct, comprising
   one or more control element(s),
   a minimal promoter,
   an inhibitory RNA coding sequence, and
   a termination sequence,

The inhibitory RNA coding sequence is selected from a coding DNA sequence for short hairpin RNA (shRNA), small interfering RNA (siRNA), micro RNA (miRNA) or antisense RNA.

The one or more control element(s) are inducible/repressible (on/off) element(s),

Step (a) is a single step which does not involve a (any) cloning step, wherein said cloning step includes the use of restriction digestion, cloning enzyme(s) as well as plasmid work, including propagation of the plasmid: bacterial transformation (transformation of plasmids into bacterial cells, culture growth) and/or plasmid preparation, plasmid extraction.

Cloning enzymes are e.g. restriction enzymes, ligase, topoisomerase, or recombinase.

Thus, the method does not require time consuming cloning and use of enzymes, for incorporating regulation sequences (such as of the TetO system).

Importantly, the expression of the inducible/repressible expression active linear RNA interference construct resulting from (a) is connected with the inducibility of the promoter.

Wherein the "inducibility" of the promoter requires the addition of a compound which activates/induces or represses the expression and/or which requires the withdrawal of a compound which represses or activates/induces the expression.

In one example described herein ("top-down" approach), step (a) comprises a PCR amplification of *a source polynucleotide* comprising the inhibitory RNA coding sequence using
(i) a forward primer comprising
   at its 3' end, a first sequence part complementary to a promoter region of the source polynucleotide upstream of the inhibitory RNA coding sequence, and
   at its 5' end, a second sequence part comprising one ore more introduced control element(s); and
(ii) a reverse primer complementary to a region of the source polynucleotide downstream of the coding region for RNA interference comprising a termination sequence,

The *source polynucleotide -* which can be used as the template providing the inhibitory RNA coding sequence - a vector (like an expression vector), a lentivirus, a plasmid, a virus-based vector, or a linear or linearized or amplified fragment thereof, or the source polynucleotide can be contained in prokaryotic or eukaryotic cells which contain inhibitory RNA coding sequences selected from sequences coding for shRNA, siRNA, miRNA or antisense RNA sequences.

The first sequence part at the 3' end of the forward primer can comprise a region of at least 6 nucleotides that binds to a region in the promoter of the source polynucleotide that is at the 5' end or upstream of the inhibitory RNA coding sequence.

In another example described herein ("bottom-up" approach) step (a) comprises a PCR amplification using
(i) a forward primer comprising
   at its 3' end, a first sequence part complementary to a promoter region of *a DNA source,* and
   at its 5' end a second sequence part comprising one ore more introduced control element(s); and
(ii) a reverse primer comprising three regions in 3' to 5' direction, namely
   a first region complementary to the 3' end of the promoter region of a DNA source,
   a second region containing the inhibitory RNA coding sequence, and a third region containing a termination sequence,

The *DNA source* can comprise at least a promoter sequence, such as a minimal promoter.

The *DNA source* can be any polynucleotide construct that harbors/comprises a promoter sequence, such as a vector (like an expression vector), a lentivirus, a plasmid, a virus-based vector, or a linear or linearized or amplified fragment thereof, or any other DNA sequence comprising the promoter sequence or the minimal promoter sequence from Polymerase I promoter, Polymerase II promoter, and Polymerase III promoter.

The methods for producing inducible and/or repressible expression active linear RNA interference constructs preferably can further comprise the following steps:
(b) transfecting a cell or cell line with the inducible/repressible expression active linear RNA interference construct obtained in step (a);
   wherein the cell or cell line transiently or stably expresses a repressor system,
(c) expressing shRNA, siRNA, miRNA or antisense RNA encoded by the inhibitory RNA coding sequence contained in the inducible/repressible expression active linear RNA interference construct obtained in step (a),
   by the addition of a compound which activates/induces or represses the expression and/or by the withdrawal of a compound which represses or activates/induces the expression;
(d) knocking out the RNA activity which is targeted by the RNA interference construct; and
(e) measuring an expression level or activity of the RNA or protein targeted by the RNA interference construct.

The minimal promoter can be derived from Pol I promoter, Pol II promoter, Pol III promoter, a synthetic minimal promoter, a viral or cellular promoter.

Pol III promoters can be U1 promoter, H1, U6, tRNA, and 7SK.
Pol II promoters can be CMV promoter, SV40, LTR, HSV TK, and ribosomal protein promoters.
Pol I promoters can be ribosomal RNA promoters.

The minimal promoter can be a minimal promoter derived from ribosomal protein promoters, such as of ribosomal protein S23 (RPS23) and of ribosomal protein S30 (RPS30), or from modified ribosomal proteins,
wherein-the RPS23 promoter or the RPS30 promoter or parts thereof or derivatives thereof can be modified for higher expression by modifying the transcriptional initiation sequence, such as by mutating a TATA-like sequence to the TATA signal sequence, such as by substituting the TATA-like signal TACAAATA with the TATA signal TATAAATA.

The termination sequence can comprise an eukaroytic polyadenylation signal, pol III termination signal, thymidines stretch, U1 termination signal, pol I termination signal, or synthetic termination variant. Throughout this application, the designation termination shall apply to the above eukaryotic signals in the embodiments.

The inducible/repressible (on/off) element(s) can be functional sequences of the tetracycline (TetO) on/off system, an ecdysone inducible system, a heat shock on/off system, a lacO/IPTG system, a cre system, a cumate repressor protein CymR system, a nitroreductase system, coumermycin/novobiocin-regulated system, a RheoSwitch Ligand RSL1 system, a chimeric bipartite nuclear receptor expression system, a GAL4 system, sterol or steroid or synthetic steroid inducing/repressing system or another inducing/repressing system or an inducing/repressing system hybrid of the above.

The inducible/repressible (on/off) element(s) can be functional sequences of the tetracycline (TetO) on/off system.

The repressor system the cell or cell line can be transiently or stably expressing (inside the cell) can be selected depending on the inducible/repressible (on/off) element(s) of the inducible/repressible expression active linear RNA interference construct.

Also, the compound, which activates or represses the expression of the inhibitory RNA sequence inside the cell or cell line and which has to be added, can be selected depending on the repressor system of the cell or cell line and the inducible/repressible (on/off) element(s) of the inducible/repressible expression active linear RNA interference construct,
and/or the compound, which represses or activates the expression of the inhibitory RNA sequence inside the cell or cell line and which has to be withdrawn, can be selected depending on the repressor system of the cell or cell line and the inducible/repressible (on/off) element(s) of the inducible/repressible expression active linear RNA interference construct.

In one example described herein the TetO system can be used, wherein
- the cell or cell line stably or transiently expresses Tet repressor or Tet activator proteins, or their mutant variants,
- the inducible/repressible (on/off) element(s) of the inducible/repressible expression active linear RNA interference construct are functional sequences of the tetracycline (TetO) on/off system;
- the compound which activates the expression of the inhibitory RNA sequence inside the cell or cell line and which has to be added is selected from tetracyclines, doxycycline, a tetracycline analogue, glycylcycline(s), a modified tetracycline, and synthetic four-ring tetracycline-like structure compounds.
Alternatively, in other systems the compound withdrawal activates the expression of the inhibitory RNA sequences.

The inhibitory RNA sequences (which are expressed) can be targeted to any gene or mRNA of interest.

The inducible/repressible expression active linear RNA interference construct can be used in animal models.

### Example concerning the first aspect described herein

In the patent application PCT/EP2008/005278, published as WO 2009/155950 A1, the inventors developed a method comprising the generation of an expression active linear reporter construct which is controllable or, in other words, regulatable or tunable. This is performed by manipulating the sequence information in the forward primers which contain two regions, a 3' end region that is directed to the vector and can be "vector position-flexible", and a 5' end region that contains *cis*-acting inducible or repressible elements.

WO 2009/155950 A1 provides a simpler cloning-free method that utilizes PCR with a specific sequence design of the forward primer along with a universal reverse primer, which will be described in details.

Described therein is a simple cloning-free PCR-based procedure to generate gene and reporter expression cassettes that can be used in many applications in the field of life sciences. This cloning-free approach allows promoter activity assessment or transcriptional manipulations including the use of *cis*-acting sequences, that otherwise require cloning and time demanding manipulations, particularly in the case of introduced mutations. In particular, a cloning-free approach to generate transcriptionally controllable linear expression active DNA is described. The expression active linear DNA produced by PCR harbors a part of a promoter, a non-inducible or minimal promoter and contains full functional gene or reporter expression cassettes that include the gene or reporter cDNA and 3'UTR. Transcriptional control elements, such as *cis*-acting elements, or their mutant forms from several bases to 140 or 200 bases are simply appended to a common DNA sequence in a forward PCR primer that targets the upstream region of the gene or reporter gene of choice, such as reporters, by a single PCR or more, if required. With two- and three-step PCRs, one can generate a 400 bases promoter or transcriptional regulatory elements, if required. The forward 5' upstream primer is versatile in its nucleotide composition, any transcriptional element or regulatory element can be added including mutations and polymorphisms. The described regulated linear reporter gene approach, i.e. introducing a transcriptional control in the linear gene PCR product is simple, versatile and adaptable to high throughput studies that are important in both academic and pharmaceutical research and development activities including drug discovery processes. The invention can be used with any different applications in the field of life sciences including, but not limited to, drug screening, drug target screening, research tool in molecular and cell biology, personalized medicine, pharmacogenomics, and correlation of genetic variations and polymorphisms with phenotypic outcomes.

The principal advantage of the invention disclosed in WO 2009/155950 A1 is the ease of introducing a transcriptional control of gene or reporter construction, ease of sequence manipulations such as mutations and deletions, reliability, and adaptability to large scale experiments and high throughput drug screening. The assay is dependent on amplification of a functional reporter expression cassette from an optimized mammalian expression vector that is efficient when transfected as PCR product to express the protein of interest. The desired promoter sequences such as a minimal promoter with or without wild type and mutant *cis-*acting sequences are included in the forward primer that contains at the 5' end several nucleotides targeting a common region upstream of the gene or reporter cDNA. The sequences in the forward primer binding to a common region in the vector or source DNA can be as little as 6 nucleotides. The preferred length is more than 10 bases, particularly preferred is more than 12 bases. The source DNA which can be used for amplification can be the vector itself, a linearized vector that contains the whole functional gene product or reporter cassette, or a linear DNA generated by PCR that contains the whole functional gene product or reporter.

The inventor has used that invention with several different strategies. All of the assays employ a universal reverse primer that target the vector DNA downstream of 3' UTR and a forward primer that targets a region upstream of the reporter construct, depending on the application. In all of the strategies, each PCR is used with one forward and one universal reverse primer.

The reverse primer is a universal primer that targets a region downstream of a polyA signal that is sufficient for optimal expression. The preferred distance from the polyA signal is at least 5 nucleotides, particularly preferred is more than 13 bases, and more preferred is more than 20 bases. The specific sequences in the universal primer binding to a vector or source DNA can be as little as 6 nucleotides. The preferred length is more than 10 bases, particularly preferred is more than 14 bases.

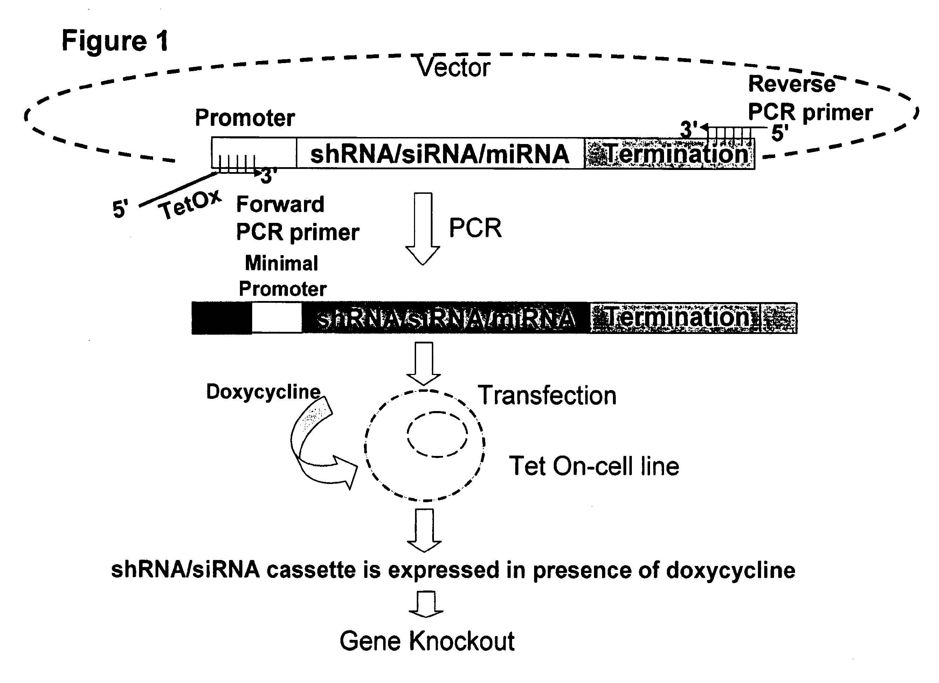

The assay is dependent on amplification of a functional gene product including a reporter expression cassette from an expression vector or DNA source that, when transfected as a linear product, is efficient to express the reporter (Fig. 1). The DNA source can be an expression vector or a fragment of the expression vector. The fragment of the expression vector should harbor the expression cassette composed of a promoter, a cDNA of the gene of interest, and a polyA signal. The vector or plasmid can be produced in abundant amounts using bacterial cultures. The fragment can be linearized by restriction fragments flanking this expression cassette. Alternatively, the expression cassette can be produced by PCR with primers flanking the expression cassette.

The primary goal of the invention of WO 2009/155950 A1 was to provide a simple method for producing transcriptional control elements and transcriptional manipulations of a reporter gene as linear DNA constructs for *"in vivo"* applications, i.e., using assays based on living cells. Specifically, the aim is to provide a simple method for manipulating promoter and transcriptional control and regulatory elements including introduced mutations and genetic variations, without the need for the time-demanding cloning steps. The method is also useful for identifying and analyzing new *cis-* and *trans*-acting regulatory sequences/factors as well as is particularly useful for drug screening and drug discovery.

The assessment and measurement of the reporter activity can be approaches, not only of the activity of the reporter proteins, but also of the levels of the reporter proteins. Reporter levels, whether intracellular or secreted, can be measured by any detection method including Western blotting, colorimetric method, fluorescence, luminescence, biosensors, and many others. Also, mRNA levels of the reporter can be used to monitor the transcription of the promoter. The mRNA levels can be assessed and quantified by a variety of techniques including, but not limited to, semi-quantitative PCR, real-time PCR, Northern blotting, RNase protection assay, beads-dependent mRNA quantification*, in situ* hybridization, and others. Examples of fluorescence, luminescence, and mRNA levels are disclosed in WO 2009/155950 A1.

Because of the ease of producing the linear reporter and introducing desired variations leading to the transcriptional control, one may expect to produce a high throughput array composed of these linear reporters harboring the different transcriptional control elements and their variations such as mutations. Nowadays, there are many high-throughput automation systems that facilitate the process including cell dispensing, transfection, and detection systems. Detection systems such as imaging of fluorescent reporters are those such as the automated imagers available from BD imaging systems (BD Dickinson, Inc.), Genetix, and Cellomics. Microplate readers and array scanners can also be applied in high throughput applications.

Thus, the method allows a versatile number of applications including, but not limited to, making a library of transcriptionally regulated functional linear reporter or gene PCR products, an array containing functional linear reporter PCR products in which each of the array feature contains a transcriptional factor or regulatory element, and a kit that contains the necessary reagents to construct the linear reporter PCR product.

Versatility of reporter systems allows use in many applications, for example, but not limited to, drug discovery, drug target discovery, bioassay development, bioassays, cytokine bioassays, interferon response bioassays, virus response bioassays, metal response bioassays, stress response bioassay, inflammatory response bioassays, cell growth assay, cellular behavior indicator assays, angiogenesis bioassay, chemotaxis and metastasis assays, hypoxia assays, environmental changes bioassays using parameters, such as heat, nutrient, radiation, oxygen, pH, salts, toxins. Additionally, any bioassay for inhibition of above responses is also a potential application.

*In the present invention,* the inventors are utilizing the method of WO 2009/155950 A1 in the exemplary embodiment of RNAi described herein.

Two important facts are directed at the source polynucleotide: (a) it harbours an inhibitory RNA coding sequence, and (b) linked to any promoter or fragment from e.g. Pol III and Pol I promoters.

"RNA interference" (RNAi) is a post-transcriptional gene-silencing mediated by homologous double-stranded dsRNA. RNAi can occur by introducing chemically synthesized siRNA or by by endogenous microRNAs (miRNA) that silence cellular mRNAs (miRNAs), reviewed in (Sledz CA, Williams BR. 2005. RNA interference in biology and disease. Blood. 106(3):787-94). The synthetic dsRNA of 21-23 nucleotides that target a specific region in the mRNA is a synthetic siRNA. Taken advantage of miRNA structure, short hairpin forms (e.g., expressed from shRNA vectors) are about 50-70 nucleotides that comprise sense strand, loop sequences, antisense strand and termination sequence. Once in cells, there hairpins are processed by dicer enzyme into an siRNA that mediates gene silencing. In mammalian cells, the antisense strand of synthetic short interfering RNA (siRNA) serves as a template for the RNA-induced silencing complex (RISC) to recognize and cleave complementary messenger RNA (mRNA), which is then rapidly degraded.

Commercially available shRNA vectors express shRNA under control of the U1 promoter. The U1 promoter is a polymerase II (Pol II) type promoter which have been used customarily for shRNA vectors. Among other useful Pol II promoters are H1, U6, tRNA, and 7SK. Also, both Pol II and Pol I promoters such as CMV promoter and ribosomal RNA promoters. The shRNA RNA vector can harbor also selection marker such hygromycin, neomycin, blasticidine or puromycin resistance gene or fluorescent markers, such as GFP, for selection of transfected cells. Any other structure of the inhibitory RNA can be used for example as miRNA based sequence. Usually shRNA consists of sense strand, loop sequence, anti-sense RNA, and termination, so that we expressed it forms a hairpin structure.

In order to convert the shRNA vector into TetO-inducible form, the shRNA vector can be used as source DNA/source polynucleotide for amplification (Figure 1). The amplified cassette includes a minimal region of the promoter that drives the expression of shRNA with incorporated TetO sequences. The incorporation of the TetO sequences was made possible by the inclusion of TetO sequence in the 5'end of the forward primer. The forward primers also has several nucleotides at the 3'end to target the desired region of the promoter The reverse primer target a region downstream of the shRNA to allow amplification of the region and inclusion of the termination sequences. The TetO sequence or its variants can be incorporated as one, two, or more copies in the forward primer.

Examplary TetO sequences are

| | |
|---|---|
| ATCCCTATCAGTGATAGA | SEQ ID NO. 9 |
| TCCCTATCAGTGATAGAGA | SEQ ID NO. 10 |
| CTATCAGTGATAGAGA | SEQ ID NO. 11 |
| TCAGTGATAGAGA | SEQ ID NO. 12 |
| or minimally TGATAG | SEQ ID NO. 13 |
| ACTCTATCATTGATAGAGT | SEQ ID NO. 14 |

or their variants or truncated variants.

The tetO-regulated system is only an example of regulated sequences that can be used. Although, tetO-based method is an exemplary embodiment and one of the most studied system and has been adopted in many several commercial vectors by cloning techniques (Gossen, M., and Bujard, H. (2002). Studying gene function in eukaryotes by conditional gene inactivation. Annual review of genetics 36, 153-173; US Patent 6,133,027). Further examples are: ecdysone inducible system, heat shock on/off system, lacO/IPTG system, cre system, cumate repressor protein CymR system, a RheoSwitch Ligand RSL1 system, coumermycin/novobiocin-regulated system, a chimeric bipartite nuclear receptor expression system, GAL4 system, nitroreductase, sterol or steroid or synthetic steroid inducing/repressing system.

Throughout the present application, the TetO sequences/system can be considered as cis-acting inducible/repressible sequences.

The method calls for converting any RNAi/shRNA vector into inducible RNAi/shRNA construct by a single PCR step without the use of plasmid-involving cloning steps or cloning enzymes, such as restriction digestion, ligase reaction, recombination, topoisomerase reaction. wherein step (a) is a single step not involving cloning steps, wherein said cloning steps include the use of restriction digestion, cloning enzymes and plasmid work.

Once the TetO-inducible RNAi/shRNA cassette is produced, it is ready to transfect desired cells. Any transfection and delivery method known in the art can be used. In Tet-on system, in the presence of doxycycline or other tetracycline analogue, the reverse tetracycline rtTA transactivator is active and binds to TetO-linked shRNA promoter activating the transcription of the shRNA resulting in knockdown of the target mRNA. In the Tet-off system, in the presence of doxycycline or tetracycline analogue, the tetracycline-controlled transactivator (tTA) is unable to bind TetO sequences and transcription is shut-off.
In tetOn-system, the shRNA is activated when induced by the tetracycline analogues, and thus, shRNA is able to knockdown the target mRNA. In Tet-off system, the shRNA is not transcribed and thus mRNA knockdown is not taking place.

### 2. Methods for assessing post-transcriptional effects or for identifying compounds that affect post-transcriptional regulation of genes, comprising producing inducible and/or repressible expression active linear gene constructs

As outlined above, the present invention provides methods for assessing post-transcriptional effects or for identifying compounds that affect post-transcriptional regulation of genes, said method comprising producing inducible and/or repressible expression active linear *gene constructs.*

The ease of the methods in converting any gene expression vector or expressable polynucleotide into TetO or other regulated constructs without virtually any cloning or plasmid preparation step is a remarkable feature of the disclosed invention.

The inducible/repressible expression active linear gene constructs are ready to be used for expressing the gene/encoded protein of interest in cells in a regulatable/regulated manner.

### - "top-down approach"

Step (a) uses as template *a source expression polynucleotide* comprising in 5' to 3' direction a promoter sequence and the DNA sequence of interest.

Said method comprises the step of:
(a) producing an inducible expression active linear gene construct, comprising
   one or more control element(s) which are inducible (on/off) elements,
   a minimal promoter which is a minimal promoter derived from a ribosomal protein promoter,
   a DNA sequence selected from a gene, a coding region, an open reading frame (ORF) and a cDNA,
   a 3' untranslated region (3' UTR) containing mRNA destabilization or stabilization elements derived from a 3' UTR of a cellular mRNA, and
   a termination sequence,
   or: (a) producing a repressible expression active linear gene construct,
   comprising
   one or more control element(s) which are repressable (on/off) elements,
   a minimal promoter which is a minimal promoter derived from a ribosomal protein promoter,
   a DNA sequence selected from a gene, a coding region, an open reading frame (ORF) and a cDNA,
   a 3' untranslated region (3' UTR) containing mRNA destabilization or stabilization elements derived from a 3' UTR of a cellular mRNA, and
   a termination sequence,
   by a PCR amplification of *a source expression polynucleotide* comprising in 5' to 3' direction a promoter sequence and the DNA sequence using
   (i) a forward primer comprising
      at its 3' end, a first sequence part complementary to a promoter region of the source expression polynucleotide upstream of the DNA sequence, and
      at its 5' end a second sequence part comprising one ore more introduced control element(s); and
   (ii) a reverse primer selected from
      a reverse primer complementary to a region of the source expression polynucleotide downstream of the DNA sequence comprising mRNA destabilization or stabilization elements derived from a 3' UTR of a cellular mRNA and a termination sequence, or
      a reverse primer complementary to a region of the source expression polynucleotide downstream of the 3' UTR wherein the region contains mRNA destabilization or stabilization elements of a cellular mRNA with or without a termination sequence,

The *source expression polynucleotide -* which is used as the template providing the DNA sequence and the promoter sequence -is selected from a vector (like an expression vector), a lentivirus, a plasmid, a virus-based vector, or a linear or linearized or amplified fragment thereof,

The minimal promoter is a minimal promoter derived from a ribosomal protein promoter, preferably of ribosomal protein S23 (RPS23) and of ribosomal protein S30 (RPS30).

Importantly, said construct is obtained in a single step which does not involve a (any) cloning step, wherein said cloning step includes the use of restriction digestion, cloning enzyme(s) as well as plasmid work, including propagation of the plasmid: bacterial transformation (transformation of plasmids into bacterial cells, culture growth) and/or plasmid preparation, plasmid extraction.

Cloning enzymes are e.g. restriction enzymes, ligase, topoisomerase, or recombinase.

Thus, the method does not require time consuming cloning and use of enzymes, for incorporating regulation sequences (such as of the TetO system).

The expression of the inducible/repressible expression active linear gene construct is connected with the inducibility of the promoter.

Wherein the "inducibility" of the promoter requires the addition of a compound which activates/induces or represses the expression and/or which requires the withdrawal of a compound which represses or activates/induces the expression.

Said method furthermore comprises the step of:
(b) assessing post-transcriptional effects at conditions that inhibit the inducible expression of the gene or protein due to the absence of a compound which activates or represses the expression and/or the presence of a compound which represses or activates the expression.

Preferably, the RPS23 promoter or the RPS30 promoter or parts thereof or derivatives thereof are modified for higher expression by modifying the transcriptional initiation sequence, preferably by mutating a TATA-like sequence to the TATA signal sequence, more preferably by substituting the TATA-like signal TACAAATA with the TATA signal TATAAATA.

Preferably the minimal promoter further comprises at least one sp1 site-containing sequence, preferably obtained by truncating the RPS23 promoter or the RPS30 promoter and adding at least one sp1 site-containing sequence.

Preferably, the minimal promoter further comprises
- intron sequence(s) of ribosomal proteins, preferably of RPS30 and/or RPS23, or parts thereof,
- exon sequence(s) of ribosomal proteins, preferably of RPS30 and/or RPS23, or parts thereof,
- modified sequences wherein the modification eliminates a restriction site.

Preferably, the minimal promoter comprises a nucleic acid sequence of any of SEQ ID NOs. 3 to 8 or a sequence complementary thereof.

Preferably, the forward primer comprises a nucleic acid sequence selected from of SEQ ID NOs. 13 or 14.

### - "bottom-up "approach

In this other example described herein, step (a) uses as template the DNA sequence of interest (such as a gene, a coding region, an open reading frame (ORF) or a cDNA) of interest, which:
- is not comprised in an expression plasmid or expression vector (it is e.g. "naked" cDNA),
- does not comprise a promoter sequence.

In this embodiment, the method for producing inducible and/or repressible expression active linear gene constructs, comprises the step of:
(a) generating an inducible/repressible expression active linear gene construct, comprising
   one or more control element(s),
   a minimal promoter,
   a DNA sequence selected from a gene, a coding region, an open reading frame (ORF) and a cDNA,
   a 3' untranslated region (3' UTR) optionally containing mRNA destabilization or stabilization elements derived from a 3' UTR of a cellular mRNA, and
   a termination sequence,
   which step comprises a PCR amplification using the DNA sequence as a template and
   (i) a forward primer comprising three regions in 5' to 3' direction, namely
      a first region containing one ore more introduced control element(s),
      a second region containing the minimal promoter,
      a third region that are at or upstream of the initiation codon of the DNA sequence, and
   (ii) a reverse primer complementary to a region at or downstream of the 3' end of the DNA sequence, with or without further comprising a termination sequence,
      optionally containing mRNA destabilization or stabilization elements derived from a 3' UTR of a cellular mRNA.

The minimal promoter comprises at least a TATA or TATA-like signal.

The one or more control element(s) are inducible/repressible (on/off) element(s).

Importantly, step (a) is a single step which does not involve a (any) cloning step, wherein said cloning step includes the use of restriction digestion, cloning enzyme(s) as well as plasmid work, including propagation of the plasmid: bacterial transformation (transformation of plasmids into bacterial cells, culture growth) and/or plasmid preparation, plasmid extraction.

Cloning enzymes are e.g. restriction enzymes, ligase, topoisomerase, or recombinase.

Thus, the method does not require time consuming cloning and use of enzymes, for incorporating regulation sequences (such as of the TetO system).

The expression of the inducible/repressible expression active linear gene construct resulting from (a) is connected with the inducibility of the promoter.

Wherein the "inducibility" of the promoter requires the addition of a compound which activates/induces or represses the expression and/or which requires the withdrawal of a compound which represses or activates/induces the expression.

This particular embodiment is preferably to convert a non-expressible DNA sequence (such as cDNA, for example from a cDNA library) that lacks a promoter, into an linear product, that can be expressed and that is also regulatable/regulated (such asTetO-regulated).

Preferably, the forward primer comprises one or more regulatory sequence element(s) or transcriptional element(s), preferably selected from transcriptional enhancing or translational enhancing element(s), such as Kozak sequence(s).

The methods for assessing post-transcriptional effects or for identifying compounds that affect post-transcriptional regulation of genes, said methods comprising producing inducible and/or repressible expression active linear gene constructs (*the top-down approach*) preferably further comprise the following steps:
(b) transfecting a cell or cell line with the inducible/repressible expression active linear gene construct obtained in step (a);
   wherein the cell or cell line transiently or stably expresses a repressor system,
(c) expressing the protein encoded by the DNA sequence contained in the inducible/repressible expression active linear gene construct obtained in step (a) by the addition of a compound which activates/induces or represses the expression and/or by the withdrawal of a compound which represses or activates/induces the expression; and
(d) measuring gene expression level or activity of the protein expressed in step (c).

The termination sequence preferably comprises an eukaroytic polyadenylation signal, pol III termination signal, thymidines stretch, U1 termination signal, pol I termination signal, or synthetic termination variant. Throughout this application, the designation termination shall apply to the above eukaryotic signals in the embodiments.

Preferably, the inducible/repressible (on/off) expression elements is selected from the group comprising functional sequences of the tetracycline (TetO) on/off system, an ecdysone inducible system, a heat shock on/off system, a lacO/IPTG system, a cre system, a cumate repressor protein CymR system, a nitroreductase system, coumermycin/novobiocin-regulated system, a RheoSwitch Ligand RSL1 system, a chimeric bipartite nuclear receptor expression system, a GAL4 system, sterol or steroid or synthetic steroid inducing/repressing system or any inducing/repressing system or hybrid system.

More preferably, the inducible/repressible (on/off) expression elements are one or more, more preferably two to three, functional sequences of the tetracycline (TetO) on/off system, such as the TetO sequence of SEQ ID NOs. 9 to 14.

An embodiment is the use of the CpG island containing sequences as inducible/repressible (on/off) expression elements to study promoter methylation activities inside cells. This embodiment can be used with any gene but preferably with a reporter. In this case, preferably, the gene or reporter is fluorescent reporter, green fluorescent protein (GFP) and derivatives, enhanced green fluorescent protein (EGFP) and derivatives, luciferase, modified luciferase, inhibitory RNA coding sequence, secreted reporter forms, alkaline phosphatase, CAT, β-galactosidase, antibody, immunoglobin fragment, cDNA fragment, and domain sequences.

The CpG islands are important in promoter function, when methylated, promoter activity is reduced. Thus, any sequence harboring CpG island and their mutants can be used to construct methylation promoter reporter system without cloning using a reporter gene. As an example, the cAMP response element TTACGTCA [SEQ ID NO. 31] and its mutant TTATGTCA-linked reporter construct [SEQ ID NO. 32] is methylated or unmethylated in cancer cells.

Preferably, the DNA sequence encodes a gene which is selected from a group comprising: reporter, fluorescent reporter, green fluorescent protein (GFP) and derivatives, enhanced green fluorescent protein (EGFP) and derivatives, luciferase, modified luciferase, inhibitory RNA coding sequence, secreted reporter forms, alkaline phosphatase, CAT, [β-galactosidase, antibody, immunoglobin fragment, cDNA fragment, open reading frame (ORF) and domain sequences.

In a preferred embodiment, the DNA sequence encodes an inhibitory RNA coding (DNA) sequence, which is selected from a coding DNA sequence for short hairpin RNA (shRNA), small interfering RNA (siRNA), micro RNA (miRNA) or antisense RNA.

It is preferred that the mRNA destabilization elements are selected from AU-rich elements, GU-rich elements, or U-rich sequences or other repeats or dinucleotides known to affect mRNA stability.
It is preferred that the mRNA stabilization elements are selected from GC-rich elements, CU-rich elements, or UG-rich sequences, or other repeats or dinucleotides known to affect mRNA stability.

Preferably, the repressor system, which the cell or cell line is transiently or stably expressing, is selected depending on the inducible/repressible (on/off) element(s) of the inducible/repressible expression active linear RNA interference construct.

Also, the compound, which activates or represses the expression of the gene or protein inside the cell or cell line and which has to be added, is selected depending on the repressor system of the cell or cell line and the inducible/repressible (on/off) element(s) of the inducible/repressible expression active linear gene construct,
and/or the compound, which represses or activates the expression of the gene or protein inside the cell or cell line and which has to be withdrawn, is selected depending on the repressor system of the cell or cell line and the inducible/repressible (on/off) element(s) of the inducible/repressible expression active linear gene construct.

### 2.1 Arrays, libraries, kits and methods utilizing the inducible/repressible expression active linear gene constructs obtained in the second method

As described above, the present invention provides the use of the inducible/repressible expression active linear gene construct as defined according to the present invention at conditions that inhibit the expression of the gene or protein for assessing post-transcriptional effects.

As described above, the present invention further provides the use of the inducible/repressible expression active linear gene construct as defined according to the present invention for identifying compounds that affect post-transcriptional regulation of genes.

The present invention further describes the use of the inducible/repressible expression active linear gene constructs for producing an array.

The array produced comprises at least two inducible/repressible expression active linear gene constructs in one or more replicate vessels.

Each vessel can contain an inducible/repressible expression active linear gene construct with a different 3'UTR or post-transcriptional control element(s).

An "array" or "microarray" refers to a multiplex technology used in molecular biology and in medicine. It consists of an arrayed series of several, many or even thousands of microscopic spots of molecules/probes (here: the constructs), called features. Typically, the molecules/probes are attached to a solid surface. The solid surface can be glass or a silicon or a plastic chip. Other microarray platforms use microscopic beads, instead of the large solid support. Arrays and microarrays are known in the art. In this application, the arrays and microarrays refer to any formats, including also 96-well plates, 384-well plates, and 1536-well plates, and higher content microarray, etc.

An array platform/format uses vessels or vessel replicates, such as in microtiter plates.

Further these arrays and microarrays may contain the constructs mixed with transfection reagents as transfection-ready format.

The present invention further describes a cell or cell line harbouring an inducible/repressible expression active linear gene construct as defined according to the present invention, such as expressing a reporter or gene protein from the construct,
wherein the cell line can be a stable cell line.

Furthermore, the present invention further provides a method for identifying compounds that affect post-transcriptional regulation of reporter(s) or gene(s).

Said method comprises the following steps
(a) providing at least one inducible/repressible expression active linear gene construct as defined according to the present invention, or a stable cell line harbouring an inducible/repressible expression active linear gene construct as defined according to the present invention expressing a reporter or gene protein from the construct,
(b) providing at least a compound to be tested,
(c) determining the effect of the compound on the post-transcriptional regulation by determining the mRNA level and/or the expression level of the reporter or gene.

Furthermore, the present invention describes a kit for carrying out the method for identifying compounds that affect post-transcriptional regulation of reporter(s) or gene(s).

Said kit can comprise
(i) at least one inducible/repressible expression active linear gene construct as defined according to the present invention,
(ii) a transfection reagent, and
(iii) an instruction sheet.

### 2.2 Minimal ribosomal promoters

As described in the application PCT/EP2008/009712, published as WO 2009/155961 A1, the inventors developed expression vectors derived from ribosomal protein 23 (RPS23) and ribosomal protein 30 (RPS30) that are transcriptionally non-inducible and constitutively active. Thus, the expression vectors are highly suitable for a number of applications, particularly for selective post-transcriptional assessment. Generally, these ribosomal protein promoters lead to weak expression levels, but, in that patent application, RPS23 and RPS30 were rendered for moderate expression. Unlike CMV and SV40 promoters, the modified RPS30IM system (wherein RPS30IM system refers to RPS30I-M1, RPS30I-M2, RPS30I-M2T, RPS30I-M1TOD as well as RPS30I-M1TOU, see below) was not activated by a number of stimuli and inducers. For example, the RPS30I-M1 system was applied to investigate responses to TNF-α or IL-α in the presence of the phosphatase inhibitor, okadaic acid, known to stabilize AU-rich elements containing-mRNAs and was found to be responsive in a manner that is independent on transcriptional induction. For more details, see herein below, and Figures and Examples.

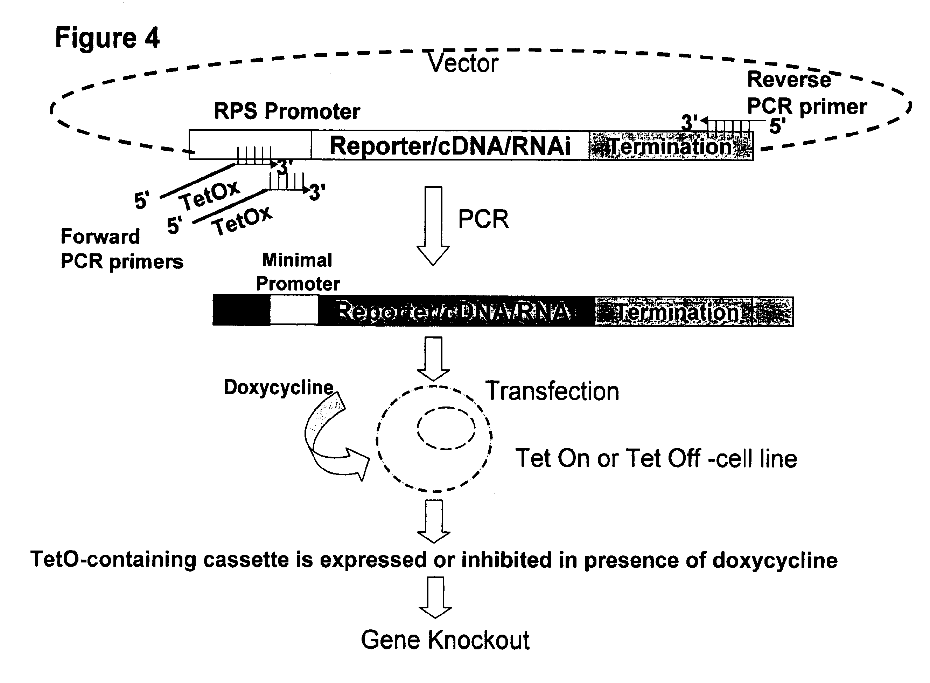

RPS23 and RPS30 and their variants (see earlier application WO 2009/155961 A1) are non-inducible cellular ribosomal promoters that can be suitable for controlled transcriptional inducibility and/or for post-transcriptional assessment. Figure 4 shows the utility of the RPS promoters and their linked polynucleotides including but not limited to cDNA, ORF, gene fragment, inhibitory RNA such as siRNA, miRNA, and shRNA coding sequences, and reporter genes. The RPS expression cassette can be used as source expression polynucleotide to generate inducible/repressible (On/Off), such as Tet-On or Tet-Off system, expression active linear gene constructs.

Preferred ribosomal protein gene promoters of the invention are the promoter of ribosomal protein S23 (RPS23) and ribosomal protein S30 (RPS30), more preferably the human RPS23 promoter or human RPS30 promoter.

The sequences of RPS23 and RPS30 can be found in the RPG ribosomal protein gene database (http://ribosome.miyazaki-med.ac.jp). See also SEQ ID NOs. 1 and 2.
The gene of *Homo sapiens* RPS30 or RPS23, respectively, contain several intron and exon sequence sections as well as a 5' upstream and a 3' downstream region, wherein the promoter region is in the 5' upstream region.

Furthermore preferred are sequences that can be derived from the preferred ribosomal protein gene promoters.

Furthermore, parts or partial sequences of the preferred ribosomal protein gene promoters are also preferred, such as truncated sequences of these promoters, e.g. 5' truncated sequences.

Preferably, truncated sequences that have a length of at least about 25 nucleotides are preferred, such as truncated sequences that have a length of about 50, 100, 150, 200, 250, 300, 350, 400 nucleotides.
In other words, the truncation is of at least 25 nucleotides or about 50 or 100 nucleotides, wherein the 5' truncation is preferred. The truncation can also be of 500, 550, 600 or more nucleotides.

Thus, truncations are preferred in a range from about 100 to 1000 nucleotides including all individual integers within that range, wherein the truncation depends on the length of the wildtype or starting sequence. The term "including all individual integers within that range", when used in relation to a range, means, for example, and when e.g. the range is 100 to 500: 100, 101, 102, 103, (...) 496, 497, 498, 500.

For example, RPS30-M1 (SEQ ID NO. 3) is 5'-truncated promoter in which 600 bases were deleted from the 5'end of the wild type promoter sequence (SEQ ID NO. 1); RPS30-M2 (SEQ ID NO. 4) is 5'-truncated promoter in which 535 bases were removed from the wild type sequence of SEQ ID NO1.

The inventors have found that these preferred promoters or parts thereof can be modified for higher expression.
The modifications are mutations, deletions, subsitutions of single or several nucleotides, insertion/including of nucleic acid sequences.

The following modifications are preferred:
- modifying the transcriptional initiation sequence,
   preferably by mutating a TATA-like sequence to the TATA signal sequence,
   more preferably by substituting the TATA-like signal TACAAATA with the TATA signal TATAAATA,
- including at least one sp1 site-containing sequence,
   preferably obtained by truncating the RPS23 promoter or the RPS30 promoter and adding at least one sp1 site-containing sequence.
   In preferred embodiments, two, three, four or more sp1 site-containing sequences are included. Sp1 sites are known in the art. Examples are given herein.

For example,
- RPS30-M1 (SEQ ID NO. 3), which is a 5'-truncated promoter in which 600 bases were deleted from the 5'end of the wild type promoter sequence (SEQ ID NO. 1), has two sp1 sites: TCCCGCCCCGTCCTGCG (position: 230-250 of SEQ ID NO. 3) and GGGGCGGAGC (position: 290-300 of SEQ ID NO. 3).
- RPS30-M2 (SEQ ID NO. 4) is a 5'-truncated promoter in which 535 bases were removed from the wild type sequence of SEQ ID NO1. A 100 bases (position of wild type) that contain additional sp1 site was added: (position of 4-21 of SEQ ID NO. 4 GCCGGGCA TGGTGGCGGG) and (position: 75-87 of SEQ ID NO. 4 GGGAGGC GGAGC). In addition to the following sp1 sites: TCCCGCCCCGTCCTGCG (position: 281-297 of SEQ ID NO. 4) and GGGGCGGAGC (position: 340-49 of SEQ ID NO. 4). Thus, RPS30-M2 contains 4 sp1 sites.

The above modifications can also be combined.

The promoter region (a) furthermore preferably comprises one or several of the following
- intron sequence(s) of ribosomal proteins, preferably of RPS30 and/or RPS 23, or parts thereof,
   preferably first intron of RPS30 (intron 1 of RPS30) or first intron of RPS23 (intron 1 of RPS23)
   The intron sequences act primarily to enhance mRNA accumulation; spliced mRNAs also exhibit higher translational yields than intron-less transcripts.
- exon sequence(s) of ribosomal proteins, preferably of RPS30 and/or RPS 23, or parts thereof,
   preferably exon 1 of RPS30 or exon 1 of RPS23
   or a part of exon 2 of RPS23 for splicing, such as the first nine nucleotides of exon 2 of RPS23,
- modified sequences wherein the modification eliminates a restriction site.
   such as
   a BamH1 site CACTGAG can be eliminated by mutating it into CACCTTGAG,
   a ATG can be eliminated by mutating it into CTG.

The expression vectors according to the present invention preferably comprise a nucleic acid sequence of any of SEQ ID NOs. 3 to 8 or a sequence complementary thereof.

### 2.3 3' untranslated regions (3' UTR)

The 3' UTR of the constructs preferably comprises or contains mRNA destabilization or stabilization elements which are derived from a 3' UTR of a cellular mRNA.

Post-transcriptional regulation can be mediated by 3' UTR that harbor mRNA destabilization elements such as AU-rich elements (for a review see Khabar and Young, 2007). Any sequence fragment of 3'UTR can be used which contains sequence elements that negatively or positively affect the post-transcriptional outcome, i.e., at mRNA or protein levels.

mRNA destabilization or stabilization elements are preferably selected from AU-rich elements, GU-rich elements, or U-rich sequences or anyother repeat sequences.

### 2. 4 TetO system

TetO is the tetracycline-resistance operon (TetO) and the TetO-based system is regulated by the repressor, rtTA, or the activator protein, tTA, the Tet-On and Tet-Off systems (Ref: Gossen M, Bujard H: Studying gene function in eukaryotes by conditional gene inactivation. Annual review of genetics 2002, 36:153-173.). The problem is that construction of TetO system requires cloning of the TetO upstream or downstream of the TATA box of a reporter or other gene product, cloning including, but not limited to use of enzymes such as restriction enzyme, topoisomearse, recombinase, and ligase and plasmid propagation is costly and time consuming particularly with high-throughput applications.

### 2. 5 Preferred embodiments of the invention

Linear constructs were generated by PCR that contains anywhere from one to more TetO copies, preferably two to three, since these are incorporated in the forward primer, and minimal ribosomal protein promoter arrangements (Figure 4).

The design of the forward primer, through 3'end sequence, allows flexibility in choosing the minimal sequences of the ribosomal protein promoter. The targeted regions that are used as examples were either -24 or -68 bases upstream the TATA box of the RPS23 or RPS30. These promoters were modified to include TATA box as they are TATA-less (see earlier application K30346PCT) DNA. The 5'end part allows inclusion of two or more copies of the TetO (SEQ 12 and 13).

The reverse primer targets a region downstream of the polyA to complete the expression active PCR product.

The PCR products are transfected into a HeLa-Tet off cell line that constitutively expresses the transactivator, tTA. In the presence of the tetracycline analog, doxycycline, tTA is inactive. In the absence of doxycycline, the tTA is active, and binds TetO containing promoter and thereby activates transcription of the linked gene.

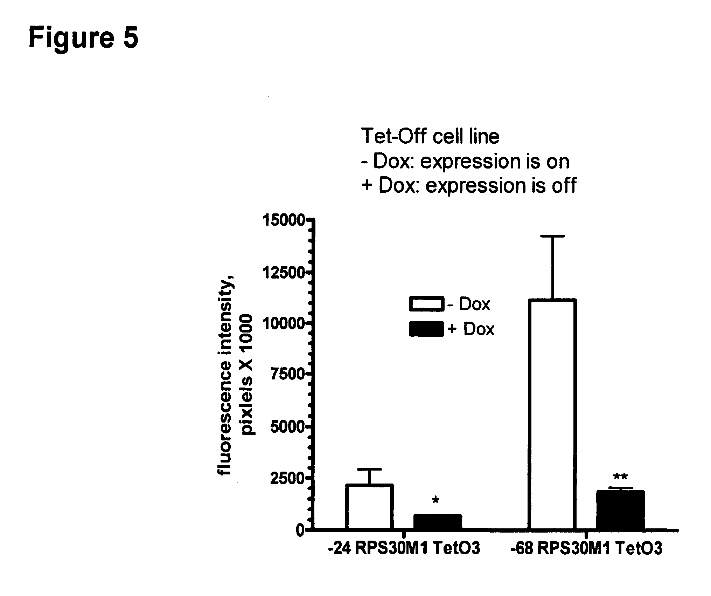

The TetO₃-minimal promoter (-24) containing construct had better suppression in the presence of doxycycline (∼87%) when compared to the construct with extended (-68) minimal promoter (Figure 5). Doxycycline has no effect on EGFP linear control constructs that lack TetO sequences.

In Tet-On system, the cells stably expresse the rtTA transactivator that is unable to bind TetO sequences in absence of doxycycline, when doxycycline is added, it activates the TetO-linked promoter.

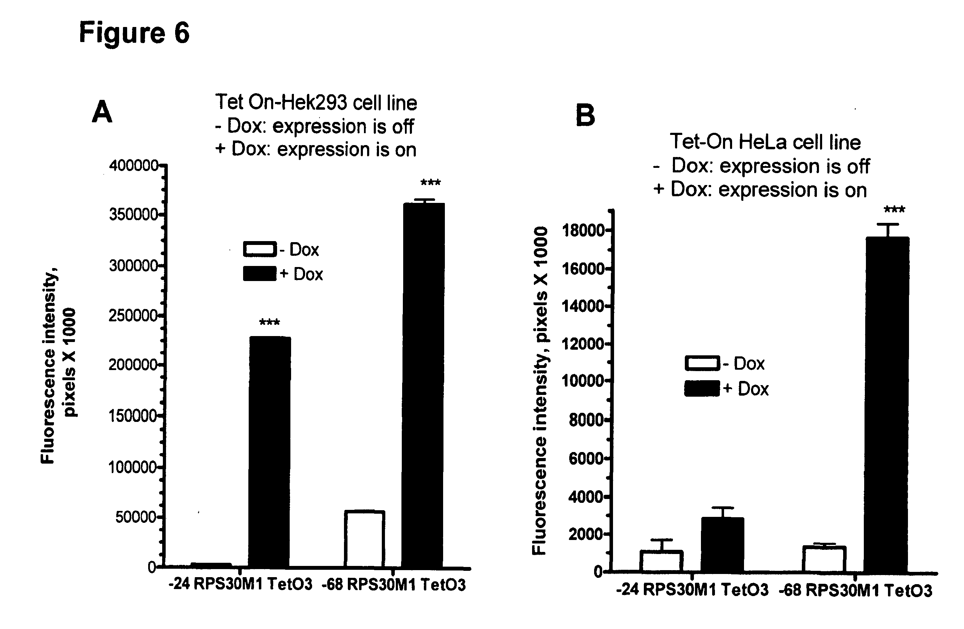

We have used both Tet-On Hek293 and Tet-Off Hela 293 (Figure 6). The -24 TetO3-linked PCR products were transfected into the cells. The -24 construct led to 77 fold induction of the linked gene in HEK293 with minimal leakage in absence of doxycycline whereas -68 construct led to more leakage and lower induction (6 -fold). Whereas, in HeLa Tet-On the -24 construct did not lead to significant induction but with the -68 construct, 13 fold induction was achieved (Figure 6).

The following drawings and examples illustrate the present invention without, however, limiting the same thereto.

### BRIEF DESCRIPTION OF THE DRAWINGS

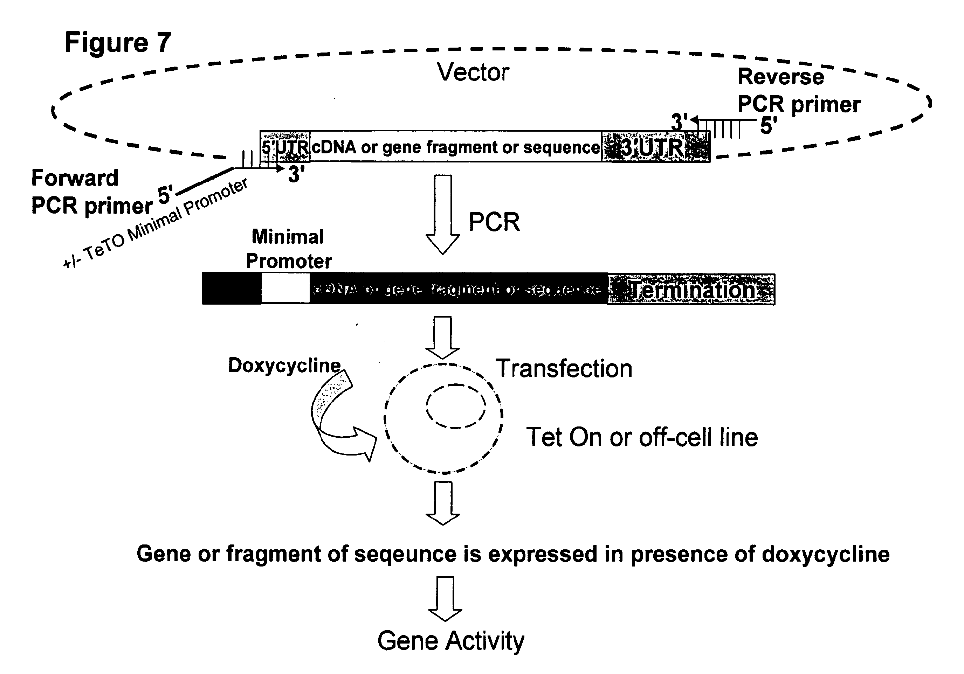

**Figure 1** *Schematic representation of conversion of shRNA or any inhibitory RNA expressing vector, such as miRNA-based vector, into tetracycline-inducible gene without cloning ("top-down method ").*
   A shRNA expression plasmid harboring shRNA that targets a gene of interest is used as a template for PCR. Forward primer contains 3' end sequences that can flexibly target a region in a promoter in the vector template to allow minimal promoter activity and 5' end sequences that contain two or three copies of TetO sites. The reverse universal primer targets a region downstream of termination site. Once transfected in a cell line that expresses the repressor that is activated when doxycycline binds to, the shRNA is transcribed and inhibits the gene activity.
**Figure 2**
   TetOn-HEK293 cell line was seeded in 96-well microplate at 3 x 10³ cell per well overnight. The cells were transfected with the TetO-regulated shRNA expressing PCR products that were obtained by converting the shRNA vector into TetO-inducible/repressible linear cassette by a single PCR method. The cells were also co-transfected with the target gene, HuR-GFP expressing plasmid (25 ng each well). Transfection was performed with lipofectinamine 2000 (Invitrogen, U.S.). After 24 hrs, the HuR protein levels were quantitated using the fluorescence output generated from the HuR-GFP fusion protein.

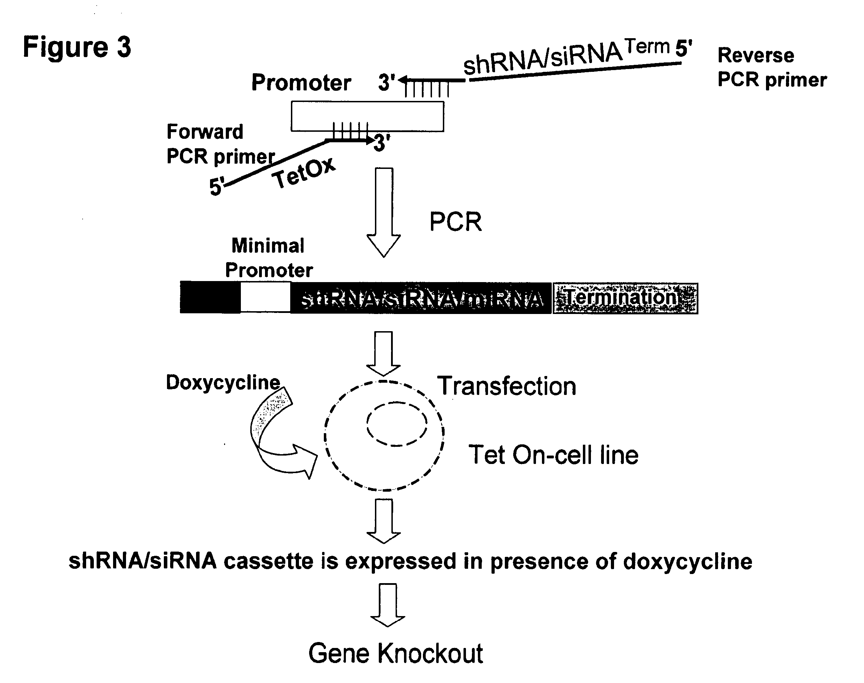

**Figure 3** *Schematic representation of generation and production of tetracycline-inducible shRNA linear construct without cloning ("bottom-up method").*
   A source polynucleotide that harbors a promoter or minimal promoter is used for PCR. Forward primer contains 3' end sequences that can flexibly target a region in a promoter in the source polynucleotide template to allow minimal promoter activity and 5'end sequences that contain two or three copies of TetO sites. The reverse universal primer is a long one that include three regions (a) a 3'end that target the 5' end of the promoter in a source polynucleotide, shRNA or siRNA or miRNA sequence, and a termination sequence. Once transfected in a cell line that expresses the repressor that is activated when doxycycline binds to, the shRNA is transcribed and inhibit the gene activity.
**Figure 4** *Schematic representation for the strategy of constructing TetO-regulated expression linear cassette using cloning-free approach without plasmid or use of enzymes.*
   Linear constructs were generated by PCR that contains anywhere from one to more TetO copies, preferably two to three, since these are incorporated in the forward primer, and minimal ribosomal protein promoter arrangements.
**Figure 5**
   The forward primer (SEQ ID NOs. 27 and 28) incorporates different arrangements of the RPS30M1 promoter regions (-24 or -68 upstream from introduced TATA box in RPS30 promoter) along with three copies of TetO. The reverse primer is downstream of the polyA termination site. The PCR products were purified using Qiagen PCR cleanup column and 75 ng products were transfected into either HEK293 Tet-Off cell line. Doxycycline was added at 1.5 µg/ml to activate shut off transcription. After 48, the fluorescence of the HuR-GFP was quantitated.
   The TetO₃-minimal promoter (-24) containing construct had better suppression in the presence of doxycycline (∼87%) when compared to the construct with extended (-68) minimal promoter.
**Figure 6** *Comparison between Tet-On Hek293 and Tet-Off Hela 293 cells.*
   We have used both Tet-On Hek293 (A) and Tet-Off Hela 293 (B). The -24 TetO3-linked PCR products were transfected into the cells. The -24 construct led to 77 fold induction of the linked gene in HEK293 with minimal leakage in absence of doxycycline whereas -68 construct led to more leakage and lower induction (6 -fold). Whereas, in HeLa Tet-On the -24 construct did not lead to significant induction but with the -68 construct, 13 fold induction was achieved
**Figure 7** *Generation of expression-active linear PCR product from naked cDNA.* Schematic representation for the strategy of constructing TetO-regulated expression linear cassette using cloning-free approach without plasmid or use of enzymes and starting from just sequence that lacks expression competency (i.e., no promoter) such as cDNA or fragment.
   Linear constructs were generated by PCR that contains anywhere from one to more TetO copies, preferably two to three, since these are incorporated in the forward primer, and minimal promoter sequence are also incorporated in the forward primer. The forward primer has also 3'end region that targets any region upstream or at the initiation codon of the cDNA or gene fragment. The reverse primer is at downstream of the cDNA 3'UTR.

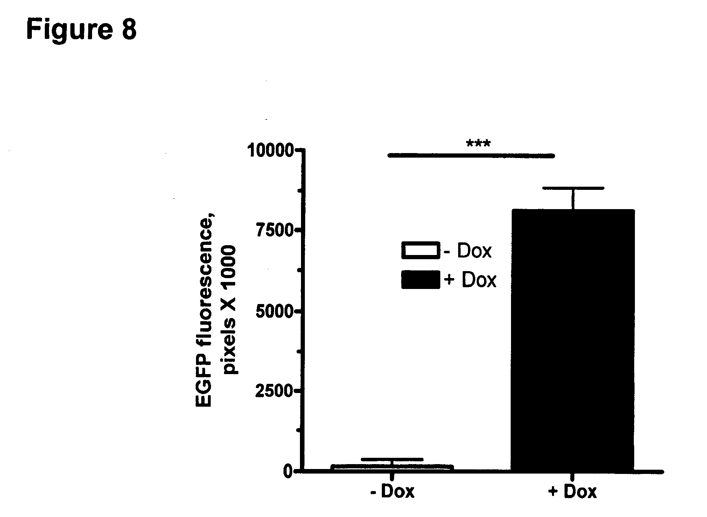

**Figure 8****.** A GFP cDNA that lacks a promoter and thus is not expressed was made expressed by amplification of the construct using a forward primers that has TATA box and other transcriptional control elements.

### EXAMPLES

### Example 1 (Comparative Example)

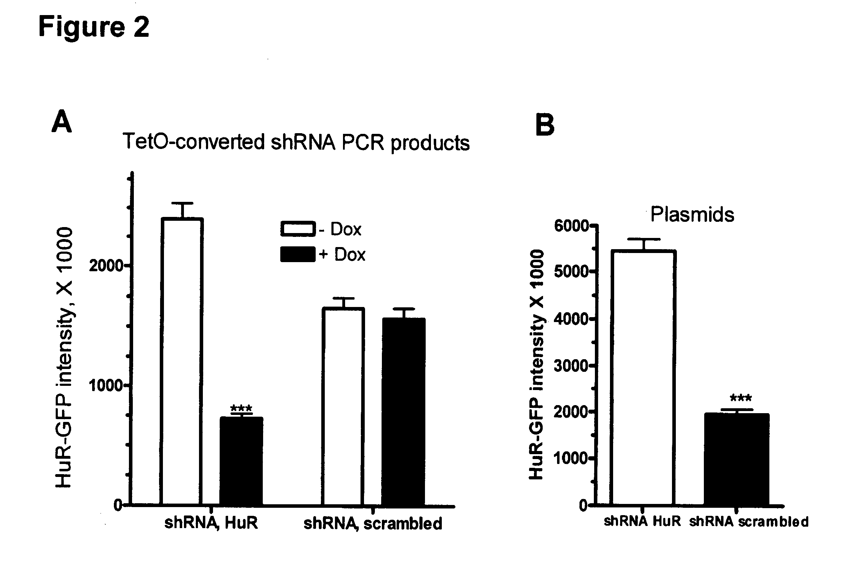

The short hairpin RNA (shRNA) vector against the RNA binding protein, HuR, was used as a source polynucleotide. This shRNA vector (Clone 4) is from SA biosciences (Maryland, U.S.) and it is under the control of U1 promoter. This shRNA vector was used as the source polynucleotide for amplifying the desired region (see Figure 1). The forward primer (SEQ ID NOs. 15 to 17) incorporates different arrangements of the U1 minimal promoter region targeted along with three copies of TetO. The reverse primer is downstream of the termination site (SEQ ID NO. 18). The PCR product was purified using Qiagen PCR cleanup column and transfected into HEK293 cells that stably express the rtTA transactivator that is unable to bind TetO sequences and activate linked promoter transcription (Clontech, Inc, CA) along with HuR-GFP fusion expression vector. After 24 hours, the fluorescence of the HuR-GFP was quantitated (see Figure 2). Similar to shRNA plasmids, the TetO-converted shRNA linear cassette was able to downregulate HuR-GFP protein levels when induced by doxycycline. Both knock down the gene activity by approximately 60% in approximately 20 hours and further knockdowns are expected with longer treatment, e.g., 70-90%.

### Example 2

The forward primer (SEQ ID NOs. 27 and 28) incorporates different arrangements of the RPS30M1 promoter regions (-24 or -68 upstream from introduced TATA box in RPS30 promoter) along with three copies of TetO. The reverse primer is downstream of the polyA termination site. The PCR products were purified using Qiagen PCR cleanup column and 75 ng products were transfected into either HEK293 Tet-Off cell line. Doxycycline was added at 1.5 µg/ml to activate shut off transcription. After 48, the fluorescence of the HuR-GFP was quantitated.

The TetO₃-minimal promoter (-24) containing construct had better suppression in the presence of doxycycline (∼87%) when compared to the construct with extended (-68) minimal promoter (see Figure 5). Doxycycline has no effect on EGFP linear control constructs that lack TetO sequences.

In Tet-On system, the cells stably expresse the rtTA transactivator that is unable to bind TetO sequences in absence of doxycycline, when doxycycline is added, it activates the TetO-linked promoter.

We have used both Tet-On Hek293 and Tet-Off Hela 293 (Figure 6). The -24 TetO3-linked PCR products were transfected into the cells. The -24 construct led to 77 fold induction of the linked gene in HEK293 with minimal leakage in absence of doxycycline whereas -68 construct led to more leakage and lower induction (6 -fold). Whereas, in HeLa Tet-On the -24 construct did not lead to significant induction but with the -68 construct, 13 fold induction was achieved (see Figure 6).

Sequences of the primers as examples:
1. U1 promoter system
   Forward primer:
   SEQ ID NO. 15 TetO3-40U1
   SEQ ID NO. 16 TetO3-80U1 SEQ ID NO. 17 TetO3-60U1
   Reverse Primer:
   SEQ ID NO. 18 U1 Termination
      CCAGTCTACTTTTGAAACTCC
2. U6 promoter system
   Forward:
   SEQ ID NO. 19 TetO3-U6
   Reverse:
   SEQ ID NO. 20 Promoter Primer
      ACGACGGCCAGTGCCAA
3. CMV promoter:
   SEQ ID NO. 21 TetO3-35
      CATCCCTATCAGTGATAGACCATCCCTATCAGTGATAGACCATCCCTATCAGTG
   SEQ ID NO. 22 TetO2-53
   SEQ ID NO. 23 HuR shR-CMVE1
   SEQ ID NO. 24 HuR shR-U1
   SEQ ID NO. 25 HuR shR-U6 (psilencer)
   SEQ ID NO. 26 HuR shRsm-CMVE1
4. RPS30
   SEQ ID NO. 27 TetO3-68TRPS30
   SEQ ID NO. 28 TetO3-24T2RPS30

### Examples 3

We have used naked cDNA that codes for GFP to convert it to expression ready cassette using PCR (Figure 7 and 8). The forward PCR primer (SEQ ID NO. 29) codes for TetO, minimal promoter, and sequence that are complementary at a region near the initiation codon of the cDNA. The reverse primer (SEQ ID NO. 30) targets a region downstream of the cDNA, i.e., downstream of the 3'UTR. When the PCR products are transfected onto cells, they express the cassette which now harbors a promoter. Any control sequence in the 5' end of the Forward primer can be used to enhance the expression.
SEQ ID NO. 29 Underlined sequence: two copies of TetO2
   Underlined italics: TATA box
   Italics: directed at the initiation codon and includes Kozac
SEQ ID NO. 30 + vector targeting sequence upstream of the cDNA
   Underlined sequence: two copies of TetO2
   Underlined italics: TATA box
   Italics: directed at the initiation codon and includes Kozac

### Ribosomal protein promoter sequences and preferred modifications

### 1. RPS30

The starting sequence that the inventors used for their promoter modifications can be found on the RPG ribosomal protein gene database (http://ribosome.miyazaki-med.ac.jp/rpg.cgi?mode=strc&id=HUM10033).
See also SEQ ID NO. 1.
The gene of *Homo sapiens* RPS30 contains several intron and exon sequence sections as well as a 5' upstream and a 3' downstream region. The putative transcriptional initiation box is tacaaata (underlined).
5' Upstream

Exon 1
   CTCTTTCTCGACTCCATCTTCGCGGTAGCTGGGACCGCCGTTCAG
Intron 1

Exon 2
Intron 2
Exon 3
Intron 3
Exon 4
   GTAAAGTCCATGGTTCCCTGGCCCGTGCTGGAAAAGTGAGAGGTCAGACTCCTAAG
Intron 4
Exon 5
3' Downstream

### 2. RPS23

The starting sequence that the inventors used for their promoter modifications can be found on the RPG ribosomal protein gene database (http://ribosome.miyazaki-med.ac.jp/rpg.cgi?mode=strc&id=HUM10025).
See also SEQ ID NO. 2.
The gene of *Homo sapiens* RPS23 contains several intron and exon sequence sections as well as a 5' upstream and a 3' downstream region.
5' Upstream
Exon 1
   CTCTTTCGCTCAGGCCCGTGGCGCCGACAGGATGG
Intron 1
Exon 2
Intron 2
Exon 3
Intron 3
Exon 4
3' Downstream

### 3. RPS30I-M1

SEQ ID NO. 3

### Modifications

1. 5' truncated RPS30 promoter (+from transcriptional start site),
2. 60 bases were added that include sp1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG.

RPS30-M1 (SEQ ID NO. 3) is a 5'-truncated promoter in which 600 bases were deleted from the 5'end of the wild type promoter sequence (SEQ ID NO. 1). RPS30-M1 has two sp1 sites: TCCCGCCCCGTCCTGCG (position 230-250) and GGGGCGGAGC (position 290-300).

### 4. RPS301-M2

SEQ ID NO. 4

### Modifications

1. 5' truncated RPS30 promoter (+from transcriptional start site),
2. 100 bases were added that include sp1 site,
3. RPS30 exon 1,
4. first intron of RPS30 , and
5. nine bases of exon 2 RPS23 for splicing.
6. /
7. BamH1 site CACTGAG was eliminated by mutating to CACCTTGAG

RPS30-M2 (SEQ ID NO. 4) is a 5'-truncated promoter in which 535 bases were removed from the wild type sequence of SEQ ID NO1. A 100 bases (position of wild type) that contain additional sp1 site was added: (position of 4-21 GCCGGGCA TGGTGGCGGG) and (position 75-87 GGGAGGC GGAGC). In addition to the following sp1 sites: TCCCGCCCCGTCCTGCG (position: 281-297) and GGGGCGGAGC (position 340-49). Thus, RPS30-M2 contains 4 sp1 sites.

### 5. RPS301-M2T

SEQ ID NO. 5

### Modifications

1. 5' truncated RPS30 promoter (+from transcriptional start site),
2. 100 bases were added that include sp1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG.

A 100 bases (position of wild type) that contain additional sp1 site was added: GCCGGGCA TGGTGGCGGG and GGGAGGC GGAGC. In addition to the following sp1 sites: TCCCGCCCCGTCCTGCG and GGGGCGGAGC. Thus, RPS30-M2T contains 4 sp1 sites.

### 6. RPS23I-M

SEQ ID NO. 6

### Modifications

1. 5' truncated promoter (+392 from transcriptional start site),
2. addition of 68 bases of Sp1 site-containing sequences of RPS23 (+806 to +872 from transcription start site),
3. RPS23 exon 1,
4. first intron of RPS23 , and
5. nine bases of exon 2 RPS23 for splicing.
6. /
7. The ATG site in exon 1 was mutated to CTG.

### 7. RPS30I-M1TOD

SEQ ID NO. 7

### Modifications

Same as RPS30I-M1 (SEQ ID NO. 3), namely
1. 5' truncated RPS30 promoter (+from transcriptional start site),
2. 60 bases were added that include sp1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG.
   in addition
8. includes tetO sequences (bold) for regulatable of transcription downstream of the TATAAA (underlined) signal.

RPS30-M1 has two sp1 sites: TCCCGCCCCGTCCTGCG and GGGGCGGAGC.

### 8. RPS30I-M1TOU

SEQ ID NO. 8

### Modifications

Same as RPS30I-M1 (SEQ ID NO. 3), namely
1. 5' truncated RPS30 promoter (+from transcriptional start site),
2. 60 bases were added that include sp1 site,
3. RPS30 exon 1,
4. first intron of RPS30 , and
5. nine bases of exon 2 RPS23 for splicing.
6. The putative transcriptional initiation box was improved from tacaaata to tataaata.
7. BamH1 site CACTGAG was eliminated by mutating to CACCTTGAG.
   in addition
8. includes tetO sequences (bold) for regulatable of transcription upstream of the TATAAA (underlined) signal.

## Claims

1. Method for assessing post-transcriptional effects or for identifying compounds that affect post-transcriptional regulation of genes, said method comprising the steps of
(a) producing an inducible expression active linear gene construct, comprising
one or more control element(s) which are inducible (on/off) element(s),
a minimal promoter which is a minimal promoter derived from a ribosomal protein promoter,
a DNA sequence selected from a gene, a coding region, an open reading frame (ORF) and a cDNA,
a 3' untranslated region (3' UTR) containing mRNA destabilization or stabilization elements derived from a 3' UTR of a cellular mRNA, and
a termination sequence,
by a PCR amplification of a source expression polynucleotide comprising in 5' to 3' direction a promoter sequence and the DNA sequence using
(i) a forward primer comprising
at its 3' end, a first sequence part complementary to a promoter region of the source expression polynucleotide upstream of the DNA sequence, and
at its 5' end a second sequence part comprising one or more introduced control element(s); and
(ii) a reverse primer selected from
a reverse primer complementary to a region of the source expression polynucleotide downstream of the DNA sequence comprising mRNA destabilization or stabilization elements derived from a 3' UTR of a cellular mRNA and a termination sequence, or
a reverse primer complementary to a region of the source expression polynucleotide downstream of the 3' UTR wherein the region contains mRNA destabilization or stabilization elements of a cellular mRNA with or without a termination sequence,
wherein the source expression polynucleotide is selected from a vector, a lentivirus, a plasmid, a virus-based vector, or a linear or linearized or amplified fragment thereof,
wherein said construct is obtained in a single step not involving a cloning step, wherein said cloning step includes the use of restriction digestion, cloning enzyme(s), bacterial transformation and/or plasmid preparation,
wherein expression of the inducible expression active linear gene construct is connected with the inducibility of the promoter, which requires the addition of a compound which activates the expression and/or which requires the withdrawal of a compound which represses the expression, and
(b) assessing post-transcriptional effects at conditions that inhibit the inducible expression of the gene or protein due to the absence of a compound which activates the expression and/or the presence of a compound which represses the expression.

2. Method for assessing post-transcriptional effects or for identifying compounds that affect post-transcriptional regulation of genes, said method comprising the steps of
(a) producing an repressible expression active linear gene construct, comprising
one or more control element(s) which are repressible (on/off) element(s),
a minimal promoter which is a minimal promoter derived from a ribosomal protein promoter,
a DNA sequence selected from a gene, a coding region, an open reading frame (ORF) and a cDNA,
a 3' untranslated region (3' UTR) containing mRNA destabilization or stabilization elements derived from a 3' UTR of a cellular mRNA, and
a termination sequence,
by a PCR amplification of a source expression polynucleotide comprising in 5' to 3' direction a promoter sequence and the DNA sequence using
(i) a forward primer comprising
at its 3' end, a first sequence part complementary to a promoter region of the source expression polynucleotide upstream of the DNA sequence, and
at its 5' end a second sequence part comprising one or more introduced control element(s); and
(ii) a reverse primer selected from
a reverse primer complementary to a region of the source expression polynucleotide downstream of the DNA sequence comprising mRNA destabilization or stabilization elements derived from a 3' UTR of a cellular mRNA and a termination sequence, or
a reverse primer complementary to a region of the source expression polynucleotide downstream of the 3' UTR wherein the region contains mRNA destabilization or stabilization elements of a cellular mRNA with or without a termination sequence,
wherein the source expression polynucleotide is selected from a vector, a lentivirus, a plasmid, a virus-based vector, or a linear or linearized or amplified fragment thereof,
wherein said construct is obtained in a single step not involving a cloning step, wherein said cloning step includes the use of restriction digestion, cloning enzyme(s), bacterial transformation and/or plasmid preparation,
wherein expression of the repressible expression active linear gene construct is connected with the inducibility of the promoter, which requires the addition of a compound which represses the expression and/or which requires the withdrawal of a compound which activates the expression, and
(b) assessing post-transcriptional effects at conditions that inhibit the inducible expression of the gene or protein due to the absence of a compound which represses the expression and/or presence of a compound which activates the expression.

3. The method of claim 1 or 2, wherein the method further comprises the following steps:
(b) transfecting a cell or cell line with the inducible/repressible expression active linear gene construct obtained in step (a);
wherein the cell or cell line transiently or stably expresses a repressor system,
(c) expressing the protein encoded by the DNA sequence contained in the inducible/repressible expression active linear gene construct
by the addition of a compound which activates or represses the expression and/or by the withdrawal of a compound which represses or activates the expression; and
(d) measuring gene expression level or activity of the protein expressed in step (c).

4. The method of any of claims 1 to 3, wherein the minimal promoter is derived from ribosomal protein S23 (RPS23) or ribosomal protein S30 (RPS30).

5. The method of claim 4, wherein the RPS23 promoter or the RPS30 promoter or parts thereof or derivatives thereof are modified for higher expression by modifying the transcriptional initiation sequence.

6. The method of claim 5, wherein the RPS23 promoter or the RPS30 promoter or parts thereof or derivatives thereof are modified by mutating a TATA-like sequence to the TATA signal sequence or by substituting the TATA-like signal TACAAATA with the TATA signal TATAAATA.

7. The method according to any of claims 1 to 6, wherein the minimal promoter further comprises at least one sp1 site-containing sequence.

8. The method of claim 7, wherein the minimal promoter further comprises at least one spl site-containing sequence obtained by truncating the RPS23 promoter or the RPS30 promoter and adding at least one sp1 site-containing sequence.

9. The method according to any of claims 1 to 8, wherein the minimal promoter further comprises
- intron sequence(s) of ribosomal proteins or parts thereof,
- exon sequence(s) of ribosomal proteins or parts thereof,
- modified sequences wherein the modification eliminates a restriction site.

10. The method of claim 9, wherein the minimal promoter further comprises intron sequence(s) of RPS30 and/or RPS23, or parts thereof.

11. The method of claim 9, wherein the minimal promoter further comprises exon sequence(s) of RPS30 and/or RPS23, or parts thereof.

12. The method according to any of the preceding claims, wherein the minimal promoter comprises a nucleic acid sequence of any of SEQ ID NOs. 3 to 8 or a sequence complementary thereof.

13. The method according to any of the preceding claims, wherein the termination sequence comprises an eukaroytic polyadenylation signal, pol III termination signal, thymidines stretch, U1 termination signal, pol I termination signal, or synthetic termination variant.

14. The method according to any of the preceding claims, wherein the inducible/repressible (on/off) expression elements is selected from the group comprising functional sequences of the tetracycline (TetO) on/off system, an ecdysone inducible system, a heat shock on/off system, a lacO/IPTG system, a cre system, a cumate repressor protein CymR system, a nitroreductase system, coumermycin/novobiocin-regulated system, a RheoSwitch Ligand RSL1 system, a chimeric bipartite nuclear receptor expression system, a GAL4 system, sterol or steroid or synthetic steroid inducing/repressing system or any inducing/repressing system or hybrid system.

15. The method of claim 14, wherein the inducible/repressible (on/off) expression elements are one or more, such as two to three, functional sequences of the tetracycline (TetO) on/off system.

16. The method of claim 14 or 15, wherein the inducible/repressible (on/off) expression elements are the TetO sequence of SEQ ID NOs. 9 to 14.

17. The method according to any of claims 1 to 14, wherein the inducible/repressible (on/off) expression elements are CpG island containing sequences.

18. The method according to any of the preceding claims, wherein the forward primer comprises a nucleic acid sequence selected from of SEQ ID NOs. 13 or 14.

19. The method according to any of the preceding claims, wherein the DNA sequence encodes a gene which is selected from a group comprising: reporter, fluorescent reporter, green fluorescent protein (GFP) and derivatives, enhanced green fluorescent protein (EGFP) and derivatives, luciferase, modified luciferase, inhibitory RNA coding sequence, secreted reporter forms, alkaline phosphatase, CAT, β-galactosidase, antibody, immunoglobin fragment, cDNA fragment, open reading frame (ORF), and domain sequences.

20. The method according to any of the preceding claims, wherein the DNA sequence encodes an inhibitory RNA coding sequence, which is selected from a coding DNA sequence for short hairpin RNA (shRNA), small interfering RNA (siRNA), micro RNA (miRNA) or antisense RNA.

21. The method according to any of the preceding claims, wherein the mRNA destabilization elements are selected from AU-rich elements, GU-rich elements, or U-rich sequences or other repeats or dinucleotides known to affect mRNA stability, and
the mRNA stabilization elements are selected from GC-rich elements, CU-rich elements, or UG-rich sequences, or other repeats or dinucleotides known to affect mRNA stability.

22. The method according to any of the preceding claims, wherein the repressor system, which the cell or cell line is transiently or stably expressing,
is selected depending on the inducible/repressible (on/off) element(s) of the inducible/repressible expression active linear RNA interference construct.

23. The method according to any of the preceding claims, wherein the compound, which activates or represses the expression of the gene or protein inside the cell or cell line and which has to be added, is selected depending on the repressor system of the cell or cell line and the inducible/repressible (on/off) element(s) of the inducible/repressible expression active linear gene construct.

24. The method according to any of the preceding claims, wherein the compound, which represses or activates the expression of the gene or protein inside the cell or cell line and which has to be withdrawn, is selected depending on the repressor system of the cell or cell line and the inducible/repressible (on/off) element(s) of the inducible/repressible- expression active linear gene construct.

25. A method for identifying compounds that affect post-transcriptional regulation of reporter(s) or gene(s), comprising the following steps
(a) providing at least one inducible/repressible expression active linear gene construct as defined in any of claims 1 to 24, or a stable cell line harbouring an inducible/repressible expression active linear gene construct as defined in any of claims 1 to 24 expressing a reporter or gene protein from the construct,
(b) providing at least a compound to be tested,
(c) determining the effect of the compound on the post-transcriptional regulation by determining the mRNA level and/or the expression level of the reporter or gene.

## Patentansprüche

1. Verfahren zur Beurteilung post-transkriptionaler Effekte oder zur Identifizierung von Verbindungen, die die post-transkriptionale Regulation von Genen beeinflussen, wobei das Verfahren die Schritte umfasst:
(a) Herstellen eines induzierbaren Expressions-aktiven linearen Genkonstrukts, umfassend
eines oder mehrere Kontrollelemente, welche induzierbare (on/off) Elemente sind,
einen minimalen Promoter, welcher ein minimaler Promoter abgeleitet von einem ribosomalen Proteinpromoter ist,
eine DNA-Sequenz ausgewählt aus einem Gen, einer kodierenden Region, einem offenen Leserahmen (ORF) und einer cDNA,
einer 3' untranslatierten Region (3' UTR) enthaltend mRNA Destabilisierungs- oder Stabilisierungselemente abgeleitet von einer 3' UTR einer zellulären mRNA, und
eine Terminationssequenz,
mittels einer PCR-Amplification eines Ausgangs-Expressionspolynukleotids umfassend in 5' zu 3' Richtung eine Promotersequenz und die DNA-Sequenz unter der Verwendung von:
(i) einem vorwärts Primer umfassend
an seinem 3'-Ende, einen ersten Sequenzteil komplementär zu einer Promoterregion des Ausgangs-Expressionspolynukleotids stromauf der DNA-Sequenz, und
an seinem 5-Ende, einen zweiten Sequenzteil umfassend eines oder mehrere eingefügte Kontrollelemente; und
(ii) einem rückwärts Primer ausgewählt aus
einem rückwärts Primer komplementär zu einer Region des Ausgangs-Expressionspolynukleotids stromab der DNA-Sequenz umfassend mRNA Destabilisierungs- oder Stabilisierungselemente abgeleitet von einer 3' UTR einer zellulären mRNA und eine Terminationssequenz, oder
einem rückwärts Primer komplementär zu einer Region des Ausgangs-Expressionspolynukleotids stromab der 3' UTR, wobei die Region mRNA Destabilisierungs- oder Stabilisierungselemente einer zellulären mRNA mit oder ohne einer Terminationssequenz enthält,
wobei das Ausgangs-Expressionspolynukleotid ausgewählt ist aus einem Vektor, einem Lentivirus, einem Plasmid, einem Virus-basierten Vektor, oder einem linearen oder linearisierten oder amplifizierten Fragment davon,
wobei das Konstrukt in einem einzigen Schritt erhalten wird, der keinen Klonierungsschritt einschließt, wobei ein Klonierungsschritt die Verwendung von Restriktionsverdau, Klonierungsenzyme(n), bakterielle Transformation und/oder Plasmidpräparation einschließt,
wobei die Expression des induzierbaren Expressions-aktiven linearen Genkonstrukts mit der Induzierbarkeit des Promoters verbunden ist, welche die Addition einer Verbindung erfordert, die die Expression aktiviert, und/oder die Entfernung einer Verbindung erfordert, die die Expression unterdrückt, und
(b) Beurteilen post-transkriptionaler Effekte bei Bedingungen, die die induzierbare Expression des Gens oder Proteins inhibieren aufgrund der Abwesenheit einer Verbindung, die die Expression aktiviert, und/oder der Anwesenheit einer Verbindung, die die Expression unterdrückt.

2. Verfahren zur Beurteilung post-transkriptionaler Effekte oder zur Identifizierung von Verbindungen, die die post-transkriptionale Regulation von Genen beeinflussen, wobei das Verfahren die Schritte umfasst:
(a) Herstellen eines unterdrückbaren Expressions-aktiven linearen Genkonstrukts, umfassend
eines oder mehrere Kontrollelemente, welche unterdrückbare (on/off) Elemente sind,
einen minimalen Promoter, welcher ein minimaler Promoter abgeleitet von einem ribosomalen Proteinpromoter ist,
eine DNA-Sequenz ausgewählt aus einem Gen, einer kodierenden Region, einem offenen Leserahmen (ORF) und einer cDNA,
einer 3' untranslatierten Region (3' UTR) enthaltend mRNA Destabilisierungs- oder Stabilisierungselemente abgeleitet von einer 3' UTR einer zellulären mRNA, und
eine Terminationssequenz,
mittels einer PCR-Amplification eines Ausgangs-Expressionspolynukleotids umfassend in 5' zu 3' Richtung eine Promotersequenz und die DNA-Sequenz unter der Verwendung von:
(i) einem vorwärts Primer umfassend
an seinem 3'-Ende, einen ersten Sequenzteil komplementär zu einer Promoterregion des Ausgangs-Expressionspolynukleotids stromauf der DNA-Sequenz, und
an seinem 5-Ende, einen zweiten Sequenzteil umfassend eines oder mehrere eingefügte Kontrollelemente; und
(ii) einem rückwärts Primer ausgewählt aus
einem rückwärts Primer komplementär zu einer Region des Ausgangs-Expressionspolynukleotids stromab der DNA-Sequenz umfassend mRNA Destabilisierungs- oder Stabilisierungselemente abgeleitet von einer 3' UTR einer zellulären mRNA und eine Terminationssequenz, oder
einem rückwärts Primer komplementär zu einer Region des Ausgangs-Expressionspolynukleotids stromab der 3' UTR, wobei die Region mRNA Destabilisierungs- oder Stabilisierungselemente einer zellulären mRNA mit oder ohne einer Terminationssequenz enthält,
wobei das Ausgangs-Expressionspolynukleotid ausgewählt ist aus einem Vektor, einem Lentivirus, einem Plasmid, einem Virus-basierten Vektor, oder einem linearen oder linearisierten oder amplifizierten Fragment davon,
wobei das Konstrukt in einem einzigen Schritt erhalten wird, der keinen Klonierungsschritt einschließt, wobei ein Klonierungsschritt die Verwendung von Restriktionsverdau, Klonierungsenzyme(n), bakterielle Transformation und/oder Plasmidpräparation einschließt,
wobei die Expression des unterdrückbaren Expressions-aktiven linearen Genkonstrukts mit der Induzierbarkeit des Promoters verbunden ist, welche die Addition einer Verbindung erfordert, die die Expression unterdrückt, und/oder die Entfernung einer Verbindung erfordert, die die Expression aktiviert, und
(b) Beurteilen post-transkriptionaler Effekte bei Bedingungen, die die induzierbare Expression des Gens oder Proteins inhibieren aufgrund der Abwesenheit einer Verbindung, die die Expression unterdrückt, und/oder der Anwesenheit einer Verbindung, die die Expression aktiviert.

3. Das Verfahren nach Anspruch 1 oder 2, wobei das Verfahren die folgenden weiteren Schritte umfasst:
(b) Transfizieren einer Zelle oder Zelllinie mit dem induzierbaren/unterdrückbaren Expressions-aktiven linearen Genkonstrukt erhalten in Schritt (a);
wobei die Zelle oder Zelllinie transient oder stabil ein Repressorsystem exprimiert,
(c) Exprimieren des Proteins kodiert von der DNA-Sequenz enthalten in dem induzierbaren/unterdrückbaren Expressions-aktiven linearen Genkonstrukt durch die Addition einer Verbindung, die die Expression aktiviert oder unterdrückt, und/oder durch das Entfernen einer Verbindung, die die Expression unterdrückt oder aktiviert; und
(d) Messen des Genexpressionslevels oder der Aktivität des Proteins exprimiert in Schritt (c).

4. Das Verfahren nach einem der Ansprüche 1 bis 3, wobei der minimale Promoter vom ribosomalen Protein S23 (RPS23) oder ribosomalen Protein S30 (RPS30) abgeleitet ist.

5. Das Verfahren nach Anspruch 4, wobei der RPS23 Promoter oder der RPS30 Promoter oder Teile davon oder Derivate davon durch Modifizieren der transkriptionalen Startsequenz für höhere Expression modifiziert ist/sind.

6. Das Verfahren nach Anspruch 5, wobei der RPS23 Promoter oder der RPS30 Promoter oder Teile davon oder Derivate davon durch Mutation einer TATA-ähnlichen Sequenz in die TATA-Signalsequenz oder durch Substitution des TATA-ähnlichen Signals TACAAATA mit der TATA-Signalsequenz TATAAATA modifiziert ist/sind.

7. Das Verfahren nach einem der Ansprüche 1 bis 6, wobei der minimale Promoter weiterhin mindestens eine sp1 Stelle-enthaltende Sequenz umfasst.

8. Das Verfahren nach Anspruch 7, wobei der minimale Promoter weiterhin mindestens eine sp1 Stelle-enthaltende Sequenz umfasst, die erhalten wird durch Trunkieren des RPS23 Promoters oder des RPS30 Promoters und durch Addition mindestens einer sp1 Stelleenthaltenden Sequenz.

9. Das Verfahren nach einem der Ansprüche 1 bis 8, wobei der minimale Promoter weiterhin umfasst
- Intronsequenz(en) von ribosomalen Proteinen, oder Teile davon,
- Exonsequenz(en) von ribosomalen Proteinen, oder Teile davon,
- modifizierte Sequenzen, wobei die Modifikation eine Restriktionsstelle eliminiert.

10. Das Verfahren nach Anspruch 9, wobei der minimale Promoter weiterhin Intronsequenz(en) von RPS30 und/oder RPS23, oder Teilen davon umfasst.

11. Das Verfahren nach Anspruch 9, wobei der minimale Promoter weiterhin Exonsequenz(en) von RPS30 und/oder RPS23, oder Teilen davon umfasst.

12. Das Verfahren nach einem der vorangehenden Ansprüche, wobei der minimale Promoter eine Nukleinsäuresequenz von einer von SEQ ID NOs. 3 bis 8 oder eine komplementäre Sequenz dazu umfasst.

13. Das Verfahren nach einem der vorangehenden Ansprüche, wobei die Terminationssequenz ein eukaryotisches Polyadenylierungssignal, pol III Terminationssignal, eine Sequenz aus mehreren Thymidinen ("thymidines stretch"), U1 Terminationssignal, pol I Terminationssignal, oder synthetische Terminationsvariante umfasst.

14. Das Verfahren nach einem der vorangehenden Ansprüche, wobei die induzierbaren/unterdrückbaren (on/off) Expressionselemente ausgewählt sind aus der Gruppe umfassend funktionale Sequenzen des Tetrazyklin (TetO) on/off System, ein Ecdysone induzierbares System, ein Hitzeschock on/off System, ein lacO/IPTG System, ein cre System, ein Cumate Repressorprotein CymR System, ein Nitroreduktase System, Coumermycin/Novobiocin-reguliertes System, ein RheoSwitch Ligand RSL1 System, ein chimäres zweiteiliges Nuklearrezeptor Expressionssystem, ein GAL4 System, Sterol oder Steroid oder synthetisches Steroid induzierbares/unterdrückbares System oder ein induzierbares/unterdrückbares System oder Hybridsystem.

15. Das Verfahren nach Anspruch 14, wobei die induzierbaren/unterdrückbaren (on/off) Expressionselemente eines oder mehrere, wie zwei bis drei, funktionale Sequenzen des Tetrazyklin (TetO) on/off Systems sind.

16. Das Verfahren nach Anspruch 14 oder 15, wobei die induzierbaren/unterdrückbaren (on/off) Expressionselemente TetO Sequenzen der SEQ ID NOs. 9 bis 14 sind.

17. Das Verfahren nach einem der Ansprüche 14, wobei die induzierbaren/unterdrückbaren (on/off) Expressionselemente CpG Inseln enthaltende Sequenzen sind.

18. Das Verfahren nach einem der vorangehenden Ansprüche, wobei der vorwärts Primer eine Nukleinsäuresequenz ausgewählt aus den SEQ ID NOs. 13 oder 14 umfasst.

19. Das Verfahren nach einem der vorangehenden Ansprüche, wobei die DNA-Sequenz ein Gen kodiert, welches ausgewählt ist aus einer Gruppe umfassend: Reporter, fluoreszierender Reporter, grün fluoreszierendes Protein (GFP) und Derivate, enhanced grün fluoreszierendes Protein (EGFP) und Derivate, Luciferase, modifizierte Luciferase, inhibitorische RNA-kodierende Sequenz, sekretierte Reporterformen, alkaline Phosphatase, CAT, β-Galactosidase, Antikörper, Immunglobinfragment, cDNA Fragment, offener Leserahmen (ORF), und Domänsequenzen.

20. Das Verfahren nach einem der vorangehenden Ansprüche, wobei die DNA-Sequenz eine inhibitorische RNA-kodierende Sequenz kodiert, welche ausgewählt ist aus einer kodierenden DNA-Sequenz für short hairpin RNA (shRNA), small interfering RNA (siRNA), micro RNA (miRNA) oder Antisense-RNA.

21. Das Verfahren nach einem der vorangehenden Ansprüche, wobei die mRNA Destabilisierungselemente ausgewählt sind aus AU-reichen Elementen, GU-reichen Elementen oder U-reichen Sequenzen oder anderen Wiederholungen ("repeats") oder Dinukleotiden, von denen bekannt ist, dass sie mRNA-Stabilität beeinflussen, und
die mRNA Stabilisierungselemente ausgewählt sind aus GC-reichen Elementen, CU-reichen Elementen oder UG-reichen Sequenzen oder anderen Wiederholungen ("repeats") oder Dinukleotiden, von denen bekannt ist, dass sie mRNA-Stabilität beeinflussen.

22. Das Verfahren nach einem der vorangehenden Ansprüche, wobei das Repressorsystem, welches die Zelle oder Zelllinie transient oder stabil exprimiert,
ausgewählt ist aus den induzierbaren/unterdrückbaren (on/off) Element(en) des induzierbaren/unterdrückbaren Expressions-aktiven linearen RNA-Interferenzkonstrukts.

23. Das Verfahren nach einem der vorangehenden Ansprüche, wobei die Verbindung, welche die Expression des Gens oder Proteins in der Zelle oder Zelllinie aktiviert oder unterdrückt und welche addiert werden muss, ausgewählt ist in Abhängigkeit vom Repressorsystem der Zelle oder Zelllinie und den induzierbaren/unterdrückbaren (on/off) Element(en) des induzierbaren/unterdrückbaren Expressions-aktiven linearen Genkonstrukts.

24. Das Verfahren nach einem der vorangehenden Ansprüche, wobei die Verbindung, welche die Expression des Gens oder Proteins in der Zelle oder Zelllinie aktiviert oder unterdrückt und welche entfernt werden muss, ausgewählt ist in Abhängigkeit vom Repressorsystem der Zelle oder Zelllinie und den induzierbaren/unterdrückbaren (on/off) Element(en) des induzierbaren/unterdrückbaren Expressions-aktiven linearen Genkonstrukts.

25. Verfahren zur Identifizierung von Verbindungen, die die post-transkriptionale Regulation von Reporter(n) oder Gen(en) beeinflussen, umfassend die folgenden Schritte:
(a) zur Verfügung stellen mindestens eines induzierbaren/unterdrückbaren Expressions-aktiven linearen Genkonstrukts, wie in einem der Ansprüche 1 bis 24 definiert, oder einer stabilen Zelllinie, die ein induzierbares/unterdrückbares Expressions-aktives lineares Genkonstrukt, wie in einem der Ansprüche 1 bis 24 definiert, enthält und einen Reporter oder ein Genprotein von dem Konstrukt exprimiert,
(b) zur Verfügung stellen mindestens einer zu testenden Verbindung,
(c) Bestimmen des Effekts der Verbindung auf die post-transkriptionale Regulation durch Bestimmen der mRNA-Level und/oder der Expressionslevel des Reporters oder Gens.

## Revendications

1. Procédé d'évaluation des effets post-transcriptionnels ou d'identification de composés qui affectent la régulation post-transcriptionnelle de gènes, ledit procédé comprenant les étapes suivantes :
(a) la production d'une construction génétique linéaire active à expression inductible, comprenant
un ou plusieurs éléments de régulation qui sont un ou plusieurs éléments inductibles (actifs/inactifs),
un promoteur minimal qui est un promoteur minimal dérivé d'un promoteur protéique ribosomique,
une séquence d'ADN choisie parmi un gène, une région codante, un cadre ouvert de lecture (ORF) et un ADNc,
une région non traduite en 3' (3' UTR) contenant des éléments de stabilisation ou de déstabilisation d'ARNm dérivés d'une 3' UTR d'un ARNm cellulaire, et
une séquence de terminaison,
par amplification par PCR d'un polynucléotide d'expression source comprenant dans le sens 5' vers 3' une séquence de promoteur et la séquence d'ADN, en utilisant
(i) une amorce sens comprenant
à son extrémité 3', une première partie de séquence complémentaire d'une région de promoteur du polynucléotide d'expression source en amont de la séquence d'ADN, et
à son extrémité 5', une seconde partie de séquence comprenant un ou plusieurs éléments de régulation introduits ; et
(ii) une amorce antisens choisie parmi
une amorce antisens complémentaire d'une région du polynucléotide d'expression source en aval de la séquence d'ADN comprenant des éléments de stabilisation ou de déstabilisation d'ARNm dérivés d'une 3' UTR d'un ARNm cellulaire et une séquence de terminaison, ou
une amorce antisens complémentaire d'une région du polynucléotide d'expression source en aval de la 3' UTR, dans laquelle la région contient des éléments de stabilisation ou de déstabilisation d'ARNm d'un ARNm cellulaire avec ou sans séquence de terminaison,
dans lequel le polynucléotide d'expression source est choisi parmi un vecteur, un lentivirus, un plasmide, un vecteur à base de virus ou un de leurs fragments linéaire ou linéarisé ou amplifié,
dans lequel ladite construction est obtenue dans une étape unique n'impliquant pas d'étape de clonage, dans laquelle ladite étape de clonage comprend l'utilisation d'une digestion par restriction, d'une ou de plusieurs enzymes de clonage, d'une transformation bactérienne et/ou d'une préparation de plasmide,
dans lequel l'expression de la construction génétique linéaire active à expression inductible est reliée à l'inductibilité du promoteur, qui requiert l'ajout d'un composé qui active l'expression et/ou qui requiert le retrait d'un composé qui réprime l'expression, et
(b) l'évaluation des effets post-transcriptionnels dans des conditions qui inhibent l'expression inductible du gène ou de la protéine en raison de l'absence d'un composé qui active l'expression et/ou de la présence d'un composé qui réprime l'expression.

2. Procédé d'évaluation des effets post-transcriptionnels ou d'identification de composés qui affectent la régulation post-transcriptionnelle de gènes, ledit procédé comprenant les étapes suivantes :
(a) la production d'une construction génétique linéaire active à expression répressible, comprenant
un ou plusieurs éléments de régulation qui sont un ou plusieurs éléments répressibles (actifs/inactifs),
un promoteur minimal qui est un promoteur minimal dérivé d'un promoteur protéique ribosomique,
une séquence d'ADN choisie parmi un gène, une région codante, un cadre ouvert de lecture (ORF) et un ADNc,
une région non traduite en 3' (3' UTR) contenant des éléments de stabilisation ou de déstabilisation d'ARNm dérivés d'une 3' UTR d'un ARNm cellulaire, et
une séquence de terminaison,
par amplification par PCR d'un polynucléotide d'expression source comprenant dans le sens 5' vers 3' une séquence de promoteur et la séquence d'ADN, en utilisant
(i) une amorce sens comprenant
à son extrémité 3', une première partie de séquence complémentaire d'une région de promoteur du polynucléotide d'expression source en amont de la séquence d'ADN, et
à son extrémité 5', une seconde partie de séquence comprenant un ou plusieurs éléments de régulation introduits ; et
(ii) une amorce antisens choisie parmi
une amorce antisens complémentaire d'une région du polynucléotide d'expression source en aval de la séquence d'ADN comprenant des éléments de stabilisation ou de déstabilisation d'ARNm dérivés d'une 3' UTR d'un ARNm cellulaire et une séquence de terminaison, ou
une amorce antisens complémentaire d'une région du polynucléotide d'expression source en aval de la 3' UTR, dans laquelle la région contient des éléments de stabilisation ou de déstabilisation d'ARNm d'un ARNm cellulaire avec ou sans séquence de terminaison,
dans lequel le polynucléotide d'expression source est choisi parmi un vecteur, un lentivirus, un plasmide, un vecteur à base de virus ou un de leurs fragments linéaire ou linéarisé ou amplifié,
dans lequel ladite construction est obtenue dans une étape unique n'impliquant pas d'étape de clonage, dans laquelle ladite étape de clonage comprend l'utilisation d'une digestion par restriction, d'une ou de plusieurs enzymes de clonage, d'une transformation bactérienne et/ou d'une préparation de plasmide,
dans lequel l'expression de la construction génétique linéaire active à expression répressible est reliée à l'inductibilité du promoteur, qui requiert l'ajout d'un composé qui réprime l'expression et/ou qui requiert le retrait d'un composé qui active l'expression, et
(b) l'évaluation des effets post-transcriptionnels dans des conditions qui inhibent l'expression inductible du gène ou de la protéine en raison de l'absence d'un composé qui réprime l'expression et/ou de la présence d'un composé qui active l'expression.

3. Procédé selon la revendication 1 ou 2, dans lequel le procédé comprend en outre les étapes suivantes :
(b) la transfection d'une cellule ou d'une lignée cellulaire avec la construction génétique linéaire active à expression inductible/répressible obtenue dans l'étape (a) ;
dans laquelle la cellule ou la lignée cellulaire exprime de manière transitoire ou stable un système répresseur,
(c) l'expression de la protéine codée par la séquence d'ADN contenue dans la construction génétique linéaire active à expression inductible/répressible
par l'ajout d'un composé qui active ou réprime l'expression et/ou par le retrait d'un composé qui réprime ou active l'expression ; et
(d) la mesure du niveau d'expression génétique ou de l'activité de la protéine exprimée dans l'étape (c).

4. Procédé selon l'une quelconque des revendications 1 à 3, dans lequel le promoteur minimal dérive de la protéine S23 ribosomique (RPS23) ou de la protéine S30 ribosomique (RPS30).

5. Procédé selon la revendication 4, dans lequel le promoteur RPS23 ou le promoteur RPS30 ou des parties de ceux-ci ou des dérivés de ceux-ci sont modifiés pour augmenter l'expression par la modification de la séquence d'initiation de la transcription.

6. Procédé selon la revendication 5, dans lequel le promoteur RPS23 ou le promoteur RPS30 ou des parties de ceux-ci ou des dérivés de ceux-ci sont modifiés par la mutation d'une séquence de type TATA en séquence de signal TATA ou par la substitution du signal de type TATA TACAAATA par le signal TATA TATAAATA.

7. Procédé selon l'une quelconque des revendications 1 à 6, dans lequel le promoteur minimal comprend en outre au moins une séquence contenant un site sp1.

8. Procédé selon la revendication 7, dans lequel le promoteur minimal comprend en outre au moins une séquence contenant un site sp1 obtenue par la troncature du promoteur RPS23 ou du promoteur RPS30 et l'ajout d'au moins une séquence contenant un site sp1.

9. Procédé selon l'une quelconque des revendications 1 à 8, dans lequel le promoteur minimal comprend en outre
- une ou plusieurs séquences d'intron de protéines ribosomiques ou des parties de celles-ci,
- une ou plusieurs séquences d'exon de protéines ribosomiques ou des parties de celles-ci,
- des séquences modifiées dans lesquelles la modification élimine un site de restriction.

10. Procédé selon la revendication 9, dans lequel le promoteur minimal comprend en outre une ou plusieurs séquences d'intron de RPS30 et/ou de RPS23 ou des parties de celles-ci.

11. Procédé selon la revendication 9, dans lequel le promoteur minimal comprend en outre une ou plusieurs séquences d'exon de RPS30 et/ou de RPS23 ou des parties de celles-ci.

12. Procédé selon l'une quelconque des revendications précédentes, dans lequel le promoteur minimal comprend l'une quelconque des séquences d'acides nucléiques représentées par SEQ ID NO. 3 à 8 ou une séquence complémentaire de celle-ci.

13. Procédé selon l'une quelconque des revendications précédentes, dans lequel la séquence de terminaison comprend un signal de polyadénylation eucaryote, un signal de terminaison pol III, une chaîne de thymidines, un signal de terminaison U1, un signal de terminaison pol I ou un variant de terminaison de synthèse.

14. Procédé selon l'une quelconque des revendications précédentes, dans lequel les éléments d'expression inductibles/répressibles (actifs/inactifs) sont choisis dans le groupe comprenant les séquences fonctionnelles du système activé/inactivé par la tétracycline (TetO), d'un système inductible par l'ecdysone, d'un système activé/désactivé par un choc thermique, d'un système lacO/IPTG, d'un système basé sur cre, d'un système basé sur le répresseur protéique CymR du cumate, d'un système basé sur la nitroréductase, d'un système régulé par la coumermycine/novobiocine, d'un système basé sur le ligand RheoSwitch RSL1, d'un système d'expression d'un récepteur nucléaire bipartite chimère, d'un système basé sur GAL4, d'un système d'induction/répression de stérol ou de stéroïde ou de stéroïde synthétique ou de n'importe quel système d'induction/répression ou système hybride.

15. Procédé selon la revendication 14, dans lequel les éléments d'expression inductibles/répressibles (actifs/inactifs) sont un ou plusieurs, telles que deux à trois, séquences fonctionnelles du système activé/inactivé par la tétracycline (TetO).

16. Procédé selon la revendication 14 ou 15, dans lequel les éléments d'expression inductibles/répressibles (actifs/inactifs) sont les séquences de TetO représentées par SEQ ID NO. 9 à 14.

17. Procédé selon l'une quelconque des revendications 1 à 14, dans lequel les éléments d'expression inductibles/répressibles (actifs/inactifs) sont des séquences contenant des îlots CpG.

18. Procédé selon l'une quelconque des revendications précédentes, dans lequel l'amorce sens comprend une séquence d'acides nucléiques choisie parmi SEQ ID NO. 13 et SEQ ID NO. 14.

19. Procédé selon l'une quelconque des revendications précédentes, dans lequel la séquence d'ADN code pour un gène qui est choisi dans le groupe comprenant : un rapporteur, un rapporteur fluorescent, la protéine fluorescente verte (GFP) et ses dérivés, la protéine fluorescente verte renforcée (EGFP) et ses dérivés, la luciférase, une luciférase modifiée, une séquence codant pour un ARN inhibiteur, des formes de rapporteur sécrété, la phosphatase alcaline, le CAT, la β-galactosidase, un anticorps, un fragment d'immunoglobuline, un fragment d'ADNc, un cadre ouvert de lecture (ORF) et des séquences de domaine.

20. Procédé selon l'une quelconque des revendications précédentes, dans lequel la séquence d'ADN code pour une séquence codant un ARN inhibiteur, qui est choisie parmi une séquence d'ADN codant pour un petit ARN en épingle à cheveux (ARNsh), un petit ARN interférent (ARNsi), un micro-ARN (miARN) ou un ARN antisens.

21. Procédé selon l'une quelconque des revendications précédentes, dans lequel les éléments de déstabilisation d'ARNm sont choisis parmi les éléments riches en AU, les éléments riches en GU ou les séquences riches en U ou d'autres répétitions ou dinucléotides connus pour affecter la stabilité des ARNm, et les éléments de stabilisation d'ARNm sont choisis parmi les éléments riches en GC, les éléments riches en CU ou les séquences riches en UG ou d'autres répétitions ou dinucléotides connus pour affecter la stabilité des ARNm.

22. Procédé selon l'une quelconque des revendications précédentes, dans lequel le système répresseur, que la cellule ou la lignée cellulaire exprime de manière transitoire ou stable,
est choisi en fonction du ou des éléments inductibles/répressibles (actifs/inactifs) de la construction linéaire active à expression inductible/répressible interférant avec un ARN.

23. Procédé selon l'une quelconque des revendications précédentes, dans lequel le composé, qui active ou réprime l'expression du gène ou de la protéine à l'intérieur de la cellule ou de la lignée cellulaire et qui doit être ajouté, est choisi en fonction du système répresseur de la cellule ou de la lignée cellulaire et du ou des éléments inductibles/répressibles (actifs/inactifs) de la construction génétique linéaire active à expression inductible/répressible.

24. Procédé selon l'une quelconque des revendications précédentes, dans lequel le composé, qui réprime ou active l'expression du gène ou de la protéine à l'intérieur de la cellule ou de la lignée cellulaire et qui doit être retiré, est choisi en fonction du système répresseur de la cellule ou de la lignée cellulaire et du ou des éléments inductibles/répressibles (actifs/inactifs) de la construction génétique linéaire active à expression inductible/répressible.

25. Procédé d'identification de composés qui affectent la régulation post-transcriptionnelle d'un ou de plusieurs rapporteurs ou d'un ou de plusieurs gènes, comprenant les étapes suivantes :
(a) la fourniture d'au moins une construction génétique linéaire active à expression inductible/répressible selon l'une quelconque des revendications 1 à 24 ou d'une lignée cellulaire stable contenant une construction génétique linéaire active à expression inductible/répressible selon l'une quelconque des revendications 1 à 24 exprimant une protéine de rapporteur ou de gène à partir de la construction,
(b) la fourniture d'au moins un composé à tester,
(c) la détermination de l'effet du composé sur la régulation post-transcriptionnelle par la détermination du taux d'ARNm et/ou du niveau d'expression du rapporteur ou du gène.


### SEQUENCE LISTING

<110> King Faisal Specialist Hospital & Research Centre
<120> Methods for producing inducible and/or repressible expression active linear RNA interference cassettes and inducible and/or repressible expression active linear gene cassettes and their uses
<130> K30352PCT
<160> 32
<170> PatentIn version 3.3
<210> 1
   <211> 3504
   <212> DNA
   <213> Homo sapiens
<400> 1
   agcgtggcct tgtttgtacc tccatgattg cctggctggc cttgctaacc taatcacatc      60
   tgtgacggga tatagtgatg tttaatctta tgattgcctt aagaattaag gcaatcagac     120
   gggttcggcg gctcatgcct gtaatcccag cactttggga ggccgaggcg ggcggatcac     180
   gaggtcagaa gatccagtcc atcctggcta acaaggtgaa accccgtctc tactaaaaat     240
   acaaaaaatt agccgggcat ggtggcggga gcctgtagtc ccagctactc gggaggctga     300
   ggcaggagga tggcgtgaat ctgggaggcg gagcttgcag tgggccgaga tcgcgccact     360
   gccctccagc ctgggcgaca gagcgagact ccgtctcaaa aaaaaaaaaa aaaagaatta     420
   aggcaatcat aattccccac gcacactcat atgctaggac cccgcccctt acctgaaacg     480
   ttgtggctta tatagacact gccaggcact gtgttaagtg ctcccaaaga gcaccccagt     540
   ctaccatttt ccctctcgat tctatatgta cactcgggac aagttctcct gatcgaaaac     600
   ggcaaaacta aggccccaag taggaatgcc ttagttttcg gggttaacaa tgattaacac      660
   tgagcctcac acccacgcga tgccctcagc tcctcgctca gcgctctcac caacagccgt     720
   agcccgcagc cccgctggac accggttctc catccccgca gcgtagcccg gaacatggta     780
   gctgccatct ttacctgcta cgccagcctt ctgtgcgcgc aactgtctgg tcccgccccg     840
   tcctgcgcga gctgcctgcc caggcaggtt cgccggtgcg agcgtaaagg ggcggagcta     900
   ggactgcctt gggcggtaca aatagcaggg aaccgcgcgg tcgctcagca gtgacgtgac     960
   acgcagccca cggtctgtac tgacgcgccc tcgcttcttc ctctttctcg actccatctt    1020
   cgcggtagct gggaccgccg ttcaggtaag aatggggcct tggctggatc cgaagggctt    1080
   gtagcaggtt ggctgcgggg tcagaaggcg cggggggaac cgaagaacgg ggcctgctcc   1140
   gtggccctgc tccagtccct atccgaactc cttgggaggc ctggccttcc ccacgtgagc    1200
   cgccgcgacc accatcccgt cgcgatcgtt tctggaccgc tttccactcc caaatctcct    1260
   ttatcccaga gcatttcttg gcttctctta caagccgtct tttctttact cagtcgccaa 1320
   tatgcagctc tttgtccgcg cccaggagct acacaccttc gaggtgaccg gccaggaaac 1380
   ggtcgcccag atcaaggtaa ggctgcttgg tgcgccctgg gttccatttt cttgtgctct 1440
   tcactctcgc ggcccgaggg aacgcttacg agccttatct ttccctgtag gctcatgtag 1500
   cctcactgga gggcattgcc ccggaagatc aagtcgtgct cctggcaggc gcgcccctgg 1560
   aggatgaggc cactctgggc cagtgcgggg tggaggccct gactaccctg gaagtagcag 1620
   gccgcatgct tggaggtgag tgagagagga atgttctttg aagtaccggt aagcgtctag 1680
   tgagtgtggg gtgcatagtc ctgacagctg agtgtcacac ctatggtaat agagtacttc 1740
   tcactgtctt cagttcagag tgattcttcc tgtttacatc cctcatgttg aacacagacg 1800
   tccatgggag actgagccag agtgtagttg tatttcagtc acatcacgag atcctagtct 1860
   ggttatcagc ttccacacta aaattaggtc agaccagggc ccccaaagtg ctctataaaa 1920
   ttagaagctg gaagatcctg aaatgaaact taagatttca aggtcaaata tctgcaactt 1980
   tgttctcatt acctattggg cgcagcttct ctttaaaggc ttgaattgag aaaagagggg 2040
   ttctgctggg tggcaccttc ttgctcttac ctgctggtgc cttcctttcc cactacaggt 2100
   aaagtccatg gttccctggc ccgtgctgga aaagtgagag gtcagactcc taaggtgagt 2160
   gagagtatta gtggtcatgg tgttaggact ttttttcctt tcacagctaa accaagtccc 2220
   tgggctctta ctcggtttgc cttctccctc cctggagatg agcctgaggg aagggatgct 2280
   aggtgtggaa gacaggaacc agggcctgat taaccttccc ttctccaggt ggccaaacag 2340
   gagaagaaga agaagaagac aggtcgggct aagcggcgga tgcagtacaa ccggcgcttt 2400
   gtcaacgttg tgcccacctt tggcaagaag aagggcccca atgccaactc ttaagtcttt 2460
   tgtaattctg gctttctcta ataaaaaagc cacttagttc agtcatcgca ttgtttcatc 2520
   tttacttgca aggcctcagg gagaggtgtg cttctcgggt tggtggtatg tcccctagga 2580
   gaacagtgag gcagaaaagg cagaagcctt tggtatgggg ggaagaaatg gtaaactaca 2640
   agagaaattt cctgtgaaga aacagctaca gatcctgggg ggcttcagat gtaaaattgg 2700
   ggttattccc tatcctaagt aacttgatca gtccccccag gtcattcttt ttcatcttct 2760
   aaacagagaa ggtagcagga atcactgtgg tgagaggttt gttatggagg cagcaataga 2820
   agggatgggt gggggaagag gtttgtatag aaggtgaacc tggccgttcc ctgaacttgg 2880
   taccagctgt ggccttagag tccagggcag gaatctggtc tgccttggtt ttagaagtaa 2940
   atattatgtt gggagcatgg cctcgtttgt acctctgtga ctgcctggcc ggacttggta 3000
   acctaatcac atctgtgatt ggatatagtg aggtttcagt gttcccaaaa gttgggttac 3060
   ctctggggct gattcagggt tctcttctgg caactgagcc tcccagcact tctgaacccc 3120
   acttactcat tcagctaaag tttctggacc tgccagttct tgagaaatag catccaacag 3180
   ggtaaagccc ttgggctgtg gactttgact gcctgagttt ggaccttctt tttcttccta   3240
   ctccatttac tgggtggctg gcctttgaac taactactaa tttaatctct gccatctcca   3300
   gggctgctgt gagggttaaa ggatgtaaat caacatctgg cttacagtga gtgtgtgaat   3360
   cttggctatt tttgtctctg tggtgttaaa gacatggttt ctgccttcca gcagtttaga   3420
   aagggggagg atgtggacag atacaatagc atcccagaga gggcctcttt ttttgttttt   3480
   ctttttcttt tattttattt tatt                                          3504
<210> 2
   <211> 4289
   <212> DNA
   <213> Homo sapiens
<400> 2
   gtctggcaca tagaaggcat ttttaaacat ccttgctgag tgaaccaata tcccagaaac     60
   ctctcacagc tagttcatct tacaggagaa acagtattaa agagttaatt aaatggccag    120
   gcgcggtggc tcacgcctgt aatcccagca cattgggagg ccgaggcggg cggatcactc    180
   gaggtcagga gatcgacacc agcctggcca acatggtgaa accccctctc tactgaaaat    240
   acaaacatca gccaggcgtg atggtggaag cctgtaatcc cagctactca ggaggctgag    300
   gtgggacaat cgcttgaacc cgggaggcgg aggttgtagt gagccaagat cgcaccactg    360
   cacaacagcc tagaagacag agtgagaccc tgtctcagaa aaaaataaaa ataaaaataa    420
   ataaatctat aagtaaatga ctcgccagtc aaaataaacg gcaactttag ggttaaaggc    480
   ccaatctggc tccaaagctt ggggttttag ttactacact acattgcttc actatatttt    540
   acaatttact agctgcttat aagtatgaat taaggctcag aagtctaatt ttccagacta    600
   ctcggaggac tctcgcccca ctccactcca caaagattca gctcagcgac tccttcctac    660
   tctgacctag ccccgcgtcc cgctctcagt ggcttgggca agagcgcctg cgcggtgagc    720
   gggtcccata aaacgcattc tgggattggt agtccatgtt cctccggtct ccagcattca    780
   aaagaaaaag ggggaaaaaa aaccatgcaa attagatatc tctgaatttc ttgcaaatta    840
   aataagacgc agattctggc tcaggaaagt gatgcaaacg cgtcgttttc aaaggagaga    900
   ccccagcctc gggtcaggcg cggcgcagac agcggcgcgg ggtccttggc tgggcggggc    960
   ttgctcgcgg tggcttgtgg ctccttcctg cggtgcttct ctctttcgct caggcccgtg   1020
   gcgccgacag gatgggtgag ctgttgtggc cggtttaagg gcgctgcaag cgggacttgg   1080
   ggtcttgggg acgggcgggc ggatgcgaat agagtagggc gggggatgcc atggagaggc   1140
   tccatggggg agggccgggg aagcgccgct ccaggaggca cgtggtccgg cgcggaaggg   1200
   gcccatgagg cgtggaggcc gccgaggtcg gggtaccgag ggacgcaggg aggccagcgc   1260
   ttcctcccgg gcattcgagc ggggcctcgt ccttcgggag aacacattct ccggagccct   1320
   cttcgaacgt ttattagtcg gttcagggca acttgaaggc caaatgtttg gcccacaggc 1380
   caataaatag tacgagagcc aatcggctta agggtttatt ccaggtgagg cgagtgtctt 1440
   agaagatggg aaacacgtag atggcgtgtt tttacggaag aactaaaata tttaattttt 1500
   aggcaagtgt cgtggacttc gtactgctag gaagctccgt agtcaccgac gagaccagaa 1560
   gtggcatgat aaacagtata agaaagctca tttgggcaca gccctaaagg ccaacccttt 1620
   tggaggtgct tctcatgcaa aaggaatcgt gctggaaaaa gtgtaagtcc attgctcccg 1680
   tcaagtttta gtttattata ggaattcgag acatgaactt acgaattctt gttttgaaag 1740
   taattgcagg tttttgtgta gtagtattca tttgggcatt gtggggtaaa attgcaaagc 1800
   gtttgttcta tttaaaagtt ggtaaaatta gtttttggga attaggtagt taaggtttta 1860
   atttaacgtt ggcctggaag gaattggaga agatactagc aatgatgaag taaaggacac 1920
   aaacaccttt actgtgggag ttgttataag taaatggcac gtgtcagcta ttgaacttta 1980
   tcgacttgat aaaactaagg tgaagagaag tgacttgcat cagaattaat tgaggtcata 2040
   cacctaagat tgagacatga aactgccagt atttgactgg ttttgacttt ttaaaataat 2100
   aatttcatat agttctatca tatttgatgg tagagccatt ttaacccaga cttttttttt 2160
   tttttttttt ttttttgaga cagtctagct ctgtcaccca ggctggtgtg cagtagcgca 2220
   agactccctg caaccttagc ctcccaggtt caagcatttc tcctgcctca gcctcccagg 2280
   tagctgggat tacaggcgcc cactaccaca ccagctaata ttttgtattt tcagtagtga 2340
   tggggtttca ccatgttgac caggctagtc tcaaactcct gacctcaggt gataatgcct 2400
   gcttcggcct ccgaaagtgc tggaattaca ggcgtgagcc actgtgtccg gcccagactt 2460
   tctaattctt acctcagata ccttttttct tttttctttt tttttttttg agatagggtc 2520
   ccttgtcaca caggctggcc atcttgacgt tctaggcata gatcctccca cgtcagcctc 2580
   gcaagtagtt gggactacag gcccacgctg ccactccagt ctacttttat aactgtaaaa 2640
   ggtctagaaa tttcccccat tgtgctaatg aaattaagac tggcagaaaa ctaggttgac 2700
   atcacaggac ttcagctcag ccatttgagg ttagattgaa aagatagaaa cagtttctca 2760
   ttagttctct agttaatatg aaaagataat ctttttcaga aagccagctc acagtgctgt 2820
   gccttttgta tttcagagga gttgaagcca aacagccaaa ttctgccatt aggaagtgtg 2880
   taagggtcca gctgatcaag aatggcaaga aaatcacagc ctttgtaccc aatgacggtt 2940
   gcttgaactt tattgaggtg agtatttcaa ctctatcgta ccttctgttc ttggggtggc 3000
   ctccctcaca tttttatctg atgcaaggga gtttcctcac atgaaagtat ttttgtgatc 3060
   gccaccaaca ccagaaataa acttcttatt ttattccagg aaaatgatga agttctggtt 3120
   gctggatttg gtcgcaaagg tcatgctgtt ggtgatattc ctggagtccg ctttaaggtt 3180
   gtcaaagtag ccaatgtttc tcttttggcc ctatacaaag gcaagaagga aagaccaaga    3240
   tcataaatat taatggtgaa aacactgtag taataaattt tcatatgcca aaaaatgttt    3300
   gtatcttact gtcccctgtt ctcaccacga agatcatgtt cattaccacc accacccccc     3360
   cttatttttt ttatcctaaa ccagcaaacg caggacctgt accaatttta ggagacaata    3420
   agacagggtt gtttcaggat tctctagagt taataacatt tgtaacctgg cacagtttcc    3480
   ctcatcctgt ggaataagaa aatgggatag atctggaata aatgtgcagt attgtagtat    3540
   tactttaaga actttaaggg aacttcaaaa actcactgaa attctagtga gatactttct    3600
   tttttattct tggtattttc catatcgggt gcaacacttc agttaccaaa tttcattgca    3660
   catagattat cttaggtacc cttggaaatg cacattcttg tatccatctt acaggggccc    3720
   aagatgataa atagtaaact caaaattgct ccccactctg tttattattt aaaggtgtca    3780
   ggatctgtgt tgtaatgtgt ctacattaat gtgtttagga gaatacaggc attggatcat    3840
   ttagttgatg gaagtatatg ccaggcaagg gagataaggt atacgacaag actgatgttt    3900
   tcagtatctt ctcatgaggt tgtcagagac cttcatgtct tcaaagacta gtcagcaaat    3960
   gaagtggttt agtgtagaga caagattggt tgtgttttga taatttaagc taggtattga    4020
   gtacatgtgg attttgctgt ccacaaatac ttgtttcaga gttttcatgg atacagtggc    4080
   atggttgaaa tgaagctgtg agccttctgc tttaaatctg atgtaagaaa ctcctgttaa    4140
   caaatagtaa gtatgggtta attagccctt tgatcaaagc ctagctttac attgtttagg    4200
   atctttggaa aacaattggt ttggttgccc actttccgta ggatcaagag cagaaccttt    4260
   cacatggcac agaagaaccc aggttgcgc                                      4289
<210> 3
   <211> 733
   <212> DNA
   <213> Artificial
<220>
   <223> modified RPS30 promoter region
<400> 3
   gatatcggca aaactaaggc cccaagtagg aatgccttag ttttcggggt taacaatgat      60
   taacactcct gagcctcaca cccacgcgat gccctcagct cctcgctcag cgctctcacc     120
   aacagccgta gcccgcagcc ccgctggaca ccgggtctcc atccccgcag cgtagcccgg     180
   aacatggtag ctgccatctt tacctgctac gccagccttc tgtgcgcgca actgtctggt     240
   cccgccccgt cctgcgcgag ctgcctgccc aggcaggttc gccggtgcga gcgtaaaggg     300
   gcggagctag gactgccttg ggcggtataa atagcaggga accgcgcggt cgctcagcag     360
   tgacgtgaca cgcagcccac ggtctgtact gacgcgccct cgcttcttcc tctttctcga     420
   ctccatcttc gcggtagctg ggaccgccgt tcaggtaaga atggggcctt ggctgcagcc     480
   gaagggcttg tagcaggttg gctgcggggt cagaaggcgc ggggggaacc gaagaacggg      540
   gcctgctccg tggccctgct ccagtcccta tccgaactcc ttgggaggcc tggccttccc     600
   cacgtgagcc gccgcgacca ccatcccgtc gcgatcgttt ctggaccgct ttccactccc     660
   aaatctcctt tatcccagag catttcttgg cttctcttac aagccgtctt ttctttactc     720
   agtcgccgtc gac                                                        733
<210> 4
   <211> 781
   <212> DNA
   <213> Artificial
<220>
   <223> modified RPS30 promoter region
<400> 4
   gatatctagc cgggcatggt ggcgggagcc tgtagtccca gctactcggg aggctgaggc      60
   aggaggatgg cgtgaatctg ggaggcggag cttgcagtgg gccgagatcg cgccacttga     120
   gcctcacacc cacgcgatgc cctcagctcc tcgctcagcg ctctcaccaa cagccgtagc     180
   ccgcagcccc gctggacacc gggtctccat ccccgcagcg tagcccggaa catggtagct     240
   gccatcttta cctgctacgc cagccttctg tgcgcgcaac tgtctggtcc cgccccgtcc     300
   tgcgcgagct gcctgcccag gcaggttcgc cggtgcgagc gtaaaggggc ggagctagga     360
   ctgccttggg cggtacaaat agcagggaac cgcgcggtcg ctcagcagtg acgtgacacg     420
   cagcccacgg tctgtactga cgcgccctcg cttcttcctc tttctcgact ccatcttcgc     480
   ggtagctggg accgccgttc aggtaagaat ggggccttgg ctgcagccga agggcttgta     540
   gcaggttggc tgcggggtca gaaggcgcgg ggggaaccga agaacggggc ctgctccgtg     600
   gccctgctcc agtccctatc cgaactcctt gggaggcctg gccttcccca cgtgagccgc     660
   cgcgaccacc atcccgtcgc gatcgtttct ggaccgcttt ccactcccaa atctccttta     720
   tcccagagca tttcttggct tctcttacaa gccgtctttt ctttactcag tcgccgtcga     780
   c                                                                     781
<210> 5
   <211> 781
   <212> DNA
   <213> Artificial
<220>
   <223> modified RPS30 promoter region
<400> 5
   gatatctagc cgggcatggt ggcgggagcc tgtagtccca gctactcggg aggctgaggc      60
   aggaggatgg cgtgaatctg ggaggcggag cttgcagtgg gccgagatcg cgccacttga     120
   gcctcacacc cacgcgatgc cctcagctcc tcgctcagcg ctctcaccaa cagccgtagc 180
   ccgcagcccc gctggacacc gggtctccat ccccgcagcg tagcccggaa catggtagct 240
   gccatcttta cctgctacgc cagccttctg tgcgcgcaac tgtctggtcc cgccccgtcc 300
   tgcgcgagct gcctgcccag gcaggttcgc cggtgcgagc gtaaaggggc ggagctagga 360
   ctgccttggg cggtataaat agcagggaac cgcgcggtcg ctcagcagtg acgtgacacg 420
   cagcccacgg tctgtactga cgcgccctcg cttcttcctc tttctcgact ccatcttcgc 480
   ggtagctggg accgccgttc aggtaagaat ggggccttgg ctgcagccga agggcttgta 540
   gcaggttggc tgcggggtca gaaggcgcgg ggggaaccga agaacggggc ctgctccgtg 600
   gccctgctcc agtccctatc cgaactcctt gggaggcctg gccttcccca cgtgagccgc 660
   cgcgaccacc atcccgtcgc gatcgtttct ggaccgcttt ccactcccaa atctccttta 720
   tcccagagca tttcttggct tctcttacaa gccgtctttt ctttactcag tcgccgtcga 780
   c 781
<210> 6
   <211> 992
   <212> DNA
   <213> Artificial
<220>
   <223> modified RPS23 promoter region
<400> 6
   gatatctggc caggcgcggt ggctcacgcc tgtaatccca gcacattggg aggccgaggc 60
   gggcggatca ctcccagact actcggagga ctctcgcccc actccactcc acaaagattc 120
   agctcagcga ctccttccta ctctgaccta gccccgcgtc ccgctctcag tggcttgggc 180
   aagagcgcct gcgcggtgag cgggtcccat aaaacgcatt ctgggattgg tagtccatgt 240
   tcctccggtc tccagcattc aaaagaaaaa gggggaaaaa aaaccatgca aattagaatc 300
   tctgaatttc ttgcaaatta aataagacgc agattctggc tcaggaaagt gatgcaaacg 360
   cgtcgttttc aaaggagaga ccccagcctc gggtcaggcg cggcgcagac agcggcgcgg 420
   ggtccttggc tgggcggggc ttgctcgcgg tggcttgtgg ctccttcctg cggtgcttct 480
   ctctttcgct caggcccgtg gcgccgacag gctgggtgag ctgttgtggc cggtttaagg 540
   gcgctgcaag cgggacttgg ggtcttgggg acgggcgggc ggatgcgaat agagtagggc 600
   gggggatgcc atggagaggc tccatggggg agggccgggg aagcgccgct ccaggaggca 660
   cgtggtccgg cgcggaaggg gcccatgagg cgtggaggcc gccgaggtcg gggtaccgag 720
   ggacgcaggg aggccagcgc ttcctcccgg gcattcgagc ggggcctcgt ccttcgggag 780
   aacacattct ccggagccct cttcgaacgt ttattagtcg gttcagggca acttgaaggc 840
   caaatgtttg gcccacaggc caataaatag tacgagagcc aatcggctaa gggtttattc 900
   caggtgaggc gagtgtctta gaagatggga aacacgtaga tggcgtgttt ttacggaaga 960
   actaaaatat ttaattttta ggcaaggtcg ac 992
<210> 7
   <211> 793
   <212> DNA
   <213> Artificial
<220>
   <223> modified RPS30 promoter region
<400> 7
   gatatcggca aaactaaggc cccaagtagg aatgccttag ttttcggggt taacaatgat 60
   taacactcct gagcctcaca cccacgcgat gccctcagct cctcgctcag cgctctcacc 120
   aacagccgta gcccgcagcc ccgctggaca ccgggtctcc atccccgcag cgtagcccgg 180
   aacatggtag ctgccatctt tacctgctac gccagccttc tgtgcgcgca actgtctggt 240
   cccgccccgt cctgcgcgag ctgcctgccc aggcaggttc gccggtgcga gcgtaaaggg 300
   gcggagctag gactgccttg ggcggtataa atagcaggga catccctatc agtgatagac 360
   catccctatc agtgatagac catccctatc agtgatagac accgcgcggt cgctcagcag 420
   tgacgtgaca cgcagcccac ggtctgtact gacgcgccct cgcttcttcc tctttctcga 480
   ctccatcttc gcggtagctg ggaccgccgt tcaggtaaga atggggcctt ggctgcagcc 540
   gaagggcttg tagcaggttg gctgcggggt cagaaggcgc ggggggaacc gaagaacggg 600
   gcctgctccg tggccctgct ccagtcccta tccgaactcc ttgggaggcc tggccttccc 660
   cacgtgagcc gccgcgacca ccatcccgtc gcgatcgttt ctggaccgct ttccactccc 720
   aaatctcctt tatcccagag catttcttgg cttctcttac aagccgtctt ttctttactc 780
   agtcgccgtc gac 793
<210> 8
   <211> 793
   <212> DNA
   <213> Artificial
<220>
   <223> modified RPS30 promoter region
<400> 8
   gatatcggca aaactaaggc cccaagtagg aatgccttag ttttcggggt taacaatgat 60
   taacactcct gagcctcaca cccacgcgat gccctcagct cctcgctcag cgctctcacc 120
   aacagccgta gcccgcagcc ccgctggaca ccgggtctcc atccccgcag cgtagcccgg 180
   aacatggtag ctgccatctt tacctgctac gccagccttc tgtgcgcgca actgtctggt 240
   cccgccccgt cctgcgcgag ctgcctgccc aggcaggttc gccggtgcga gcgtaaaggg 300
   gcatccctat cagtgataga ccatccctat cagtgataga ccatccctat cagtgataga 360
   ccggagctag gactgccttg ggcggtataa atagcaggga accgcgcggt cgctcagcag 420
   tgacgtgaca cgcagcccac ggtctgtact gacgcgccct cgcttcttcc tctttctcga 480
   ctccatcttc gcggtagctg ggaccgccgt tcaggtaaga atggggcctt ggctgcagcc 540
   gaagggcttg tagcaggttg gctgcggggt cagaaggcgc ggggggaacc gaagaacggg 600
   gcctgctccg tggccctgct ccagtcccta tccgaactcc ttgggaggcc tggccttccc 660
   cacgtgagcc gccgcgacca ccatcccgtc gcgatcgttt ctggaccgct ttccactccc 720
   aaatctcctt tatcccagag catttcttgg cttctcttac aagccgtctt ttctttactc 780
   agtcgccgtc gac 793
<210> 9
   <211> 18
   <212> DNA
   <213> Artificial
<220>
   <223> TetO sequence
<400> 9
   atccctatca gtgataga 18
<210> 10
   <211> 19
   <212> DNA
   <213> Artificial
<220>
   <223> TetO sequence
<400> 10
   tccctatcag tgatagaga 19
<210> 11
   <211> 16
   <212> DNA
   <213> Artificial
<220>
   <223> TetO sequence
<400> 11
   ctatcagtga tagaga 16
<210> 12
   <211> 13
   <212> DNA
   <213> Artificial
<220>
   <223> TetO sequence
<400> 12
   tcagtgatag aga 13
<210> 13
   <211> 6
   <212> DNA
   <213> Artificial
<220>
   <223> minimal TetO sequence
<400> 13
   tgatag 6
<210> 14
   <211> 19
   <212> DNA
   <213> Artificial
<220>
   <223> TetO sequence
<400> 14
   actctatcat tgatagagt 19
<210> 15
   <211> 75
   <212> DNA
   <213> Artificial
<220>
   <223> Forward primer
<400> 15
   catccctatc agtgatagac catccctatc agtgatagac catccctatc agtgatgagg 60
   cgtatgaggc tgtgt 75
<210> 16
   <211> 74
   <212> DNA
   <213> Artificial
<220>
   <223> Forward primer
<400> 16
   catccctatc agtgatagac catccctatc agtgatagac catccctatc agtgatagtg 60
   cgcggggcaa gtga 74
<210> 17
   <211> 77
   <212> DNA
   <213> Artificial
<220>
   <223> Forward primer
<400> 17
   catccctatc agtgatagac catccctatc agtgatagac catccctatc agtgatgtga 60
   ccgtgtgtgt aaagagt 77
<210> 18
   <211> 21
   <212> DNA
   <213> Artificial
<220>
   <223> Reverse primer
<400> 18
   ccagtctact tttgaaactc c 21
<210> 19
   <211> 77
   <212> DNA
   <213> Artificial
<220>
   <223> Forward primer
<400> 19
   catccctatc agtgatagac catccctatc agtgatagac catccctatc agtgatggac 60
   tatcatatgc ttaccgt 77
<210> 20
   <211> 17
   <212> DNA
   <213> Artificial
<220>
   <223> Reverse primer
<400> 20
   acgacggcca gtgccaa 17
<210> 21
   <211> 54
   <212> DNA
   <213> Artificial
<220>
   <223> Primer
<400> 21
   catccctatc agtgatagac catccctatc agtgatagac catccctatc agtg 54
<210> 22
   <211> 64
   <212> DNA
   <213> Artificial
<220>
   <223> Primer
<400> 22
   accaggtccc tatcagtgat agagatcctc cctatcagtg atagagaggt aggcgtgtac 60
   ggtg 64
<210> 23
   <211> 76
   <212> DNA
   <213> Artificial
<220>
   <223> Primer
<400> 23
   gaaaaaagaa gaggcaatta ccagttttga caggaagaaa ctggtaattg cctcttctcg 60
   gttcactaaa cgagct 76
<210> 24
   <211> 76
   <212> DNA
   <213> Artificial
<220>
   <223> Primer
<400> 24
   gaaaaaagaa gaggcaatta ccagttttga caggaagaaa ctggtaattg cctcttctcg 60
   agatcttggg cctctg 76
<210> 25
   <211> 72
   <212> DNA
   <213> Artificial
<220>
   <223> Primer
<400> 25
   aaaagaagag gcaattacca gttttgacag gaagaaactg gtaattgcct cttctcgcgt 60
   cctttccaca ag 72
<210> 26
   <211> 67
   <212> DNA
   <213> Artificial
<220>
   <223> Primer
<400> 26
   aagaagaggc aattaccagt ttctcttgaa aactggtaat tgcctcttcc ggttcactaa 60
   acgagct 67
<210> 27
   <211> 81
   <212> DNA
   <213> Artificial
<220>
   <223> Forward primer
<400> 27
   catccctatc agtgatagac catccctatc agtgatagac catccctatc agtgatagac 60
   gctgcctgcc caggcaggtt c 81
<210> 28
   <211> 80
   <212> DNA
   <213> Artificial
<220>
   <223> Forward primer
<400> 28
   catccctatc agtgatagac catccctatc agtgatagac catccctatc agtgatagac 60
   ggcggagcta ggactgcctt 80
<210> 29
   <211> 82
   <212> DNA
   <213> Artificial
<220>
   <223> Forward primer
<400> 29
   accaggtccc tatcagtgat agagatcctc cctatcagtg atagagactg ggtatataat 60
   ggaagccacc atggccagca ag 82
<210> 30
   <211> 64
   <212> DNA
   <213> Artificial
<220>
   <223> Reverse primer
<400> 30
   accaggtccc tatcagtgat agagatcctc cctatcagtg atagagactg ggtatataat 60
   ggaa 64
<210> 31
   <211> 8
   <212> DNA
   <213> Artificial
<220>
   <223> cAMP response element
<400> 31
   ttacgtca 8
<210> 32
   <211> 8
   <212> DNA
   <213> Artificial
<220>
   <223> cAMP response element
<400> 32
   ttatgtca 8